(19) Europäisches Patentamt
European Patent Office
Office européen des brevets

(11) Publication number: **0 271 170 A2**

(12) **EUROPEAN PATENT APPLICATION**

(21) Application number: **87202464.1**

(22) Date of filing: **08.12.87**

(51) Int. Cl.[4]: **C07D 405/04**, C07D 471/04, A01N 43/00, C07D 209/48

(30) Priority: **10.12.86 US 943365**

(43) Date of publication of application: **15.06.88 Bulletin 88/24**

(84) Designated Contracting States: **AT BE CH DE ES FR GB GR IT LI LU NL SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Semple, Joseph Edward**
**436 Coldspring Run**
**Newark, DE 19711(US)**

(74) Representative: **Hunter, Keith Roger Ian et al**
**4 York Road**
**London SE1 7NA(GB)**

(54) **Herbicidal substituted benzofurans and benzopyrans.**

(57) Herbicidal N-(heterocyclyl)-substitued benzofurans and benzopyrans of the formulae:

EP 0 271 170 A2

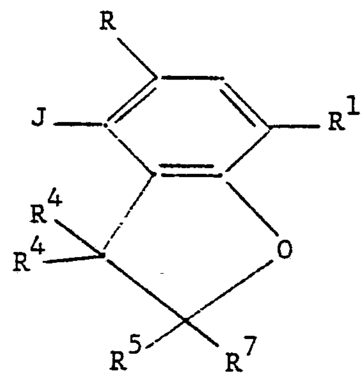

A

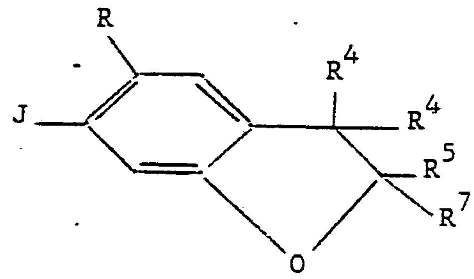

B

(I)

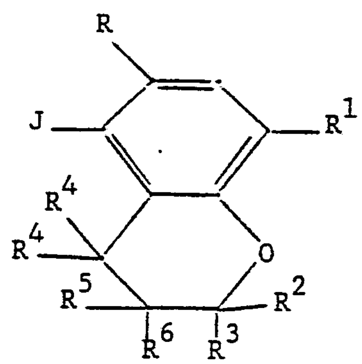

C

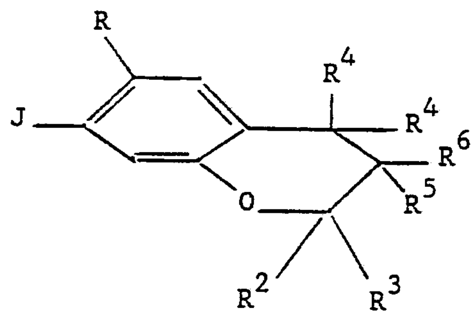

D

wherein the symbols have meanings described in the specification.

87202464.1

K 3609 FF

# HERBICIDAL SUBSTITUTED BENZOFURANS AND BENZOPYRANS

This invention relates to certain N-heterocyclic-substituted benzofurans and benzopyrans, their preparation, herbicidal compositions comprising these compounds and their use as herbicides. The invention also includes novel intermediates in the preparation of the benzofurans and benzopyrans.

The invention relates to compounds of the formulae I:

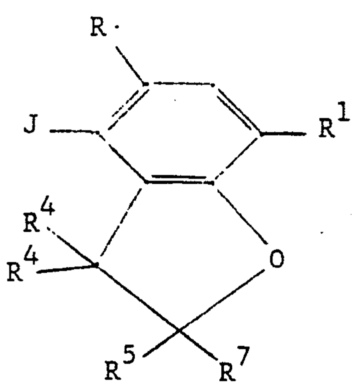

A

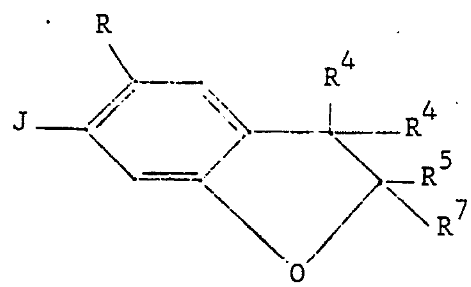

B

(I)

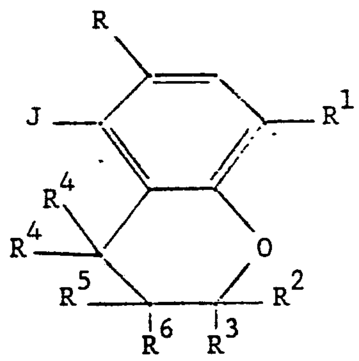

C

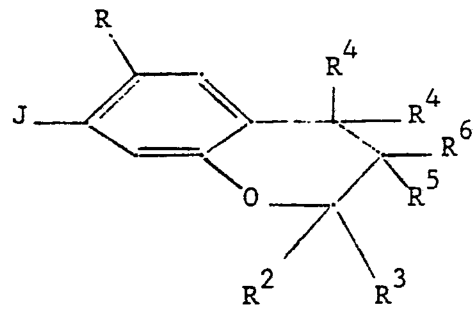

D

wherein

R and $R^1$ each independently represents a hydrogen or a halogen atom; a cyano group; an alkyl or alkoxy group having one to four carbon atoms; a trihalomethyl group; a hydroxymethyl group; an alkyl-, alkenyl-, or alkanoyl-oxymethyl group having up to four carbon atoms; or $-OCF_nH_{3-n}$ where n is one, two or three;

each of $R^4$ is independently a hydrogen atom or an alkyl group having one to four carbon atoms;

$R^5$ represents a hydrogen atom or an alkyl group having one to four carbon atoms;

$R^6$ is a hydrogen atom or a hydroxy group; with the proviso that $R^5$ and $R^6$ together can be an oxo (=O) oxygen atom or a moiety $=NO(R^8)$; and with the further proviso that when $R^5$ is a hydrogen atom, $R^6$ is -CN, -C(O)Cl, $-C(O)R^8$, $-C(O)O(R^8)$, or $-C(O)N(R^8)(R^8)$;

$R^7$ represents a group -CN, $-CH(R^9)CN$, $-CH(R^2)(R^3)$, -C(O)Cl, $-C(O)R^8$, $-C(O)O(R^8)$, $-CH(R^9)-O(R^{10})$, $-C(O)N(R^8)(R^8)$ or $-C(R^9)=NO(R^8)$, wherein $R^2$ and $R^3$ each independently represents a hydrogen atom; an alkyl or haloalkyl group of one to four carbon atoms; a phenyl group; or a phenylalkyl group having seven to ten carbon atoms;

$R^8$ represents a hydrogen atom; an alkyl, haloalkyl, alkenyl, haloalkenyl or alkynyl group of up to four carbon atoms; a phenyl or phenylalkyl group having seven to ten carbon

atoms; a heteroaryl or heteroaralkyl group having up to ten carbon atoms; or an alkoxyalkyl or alkoxycarbonylalkyl group having up to six carbon atoms;

$R^9$ represents a hydrogen atom or an alkyl group having one to four carbon atoms;

$R^{10}$ represents a hydrogen atom; an alkyl, haloalkyl, alkenyl, haloalkenyl or alkynyl group having up to four carbon atoms; a phenylalkyl group having seven to ten carbon atoms; a heteroaralkyl group having up to ten carbon atoms; or each of the foregoing bonded to a carbonyl moiety that is bonded to the oxygen atom; an alkoxyalkyl or alkoxycarbonylalkyl group having up to six carbon atoms; an alkylsulfonyl group having one to four carbon atoms; a phenylsulfonyl group which may be substituted on the ring by one or more halogen atoms and/or alkyl groups having one to four carbon atoms; a carbamoyl group, or an alkyl- or dialkylcarbamoyl group having up to six carbon atoms;

and J is one of the moieties:

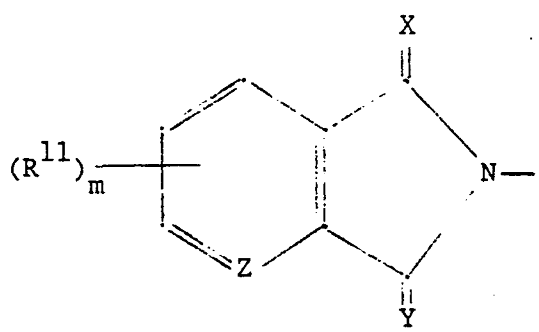

J-1

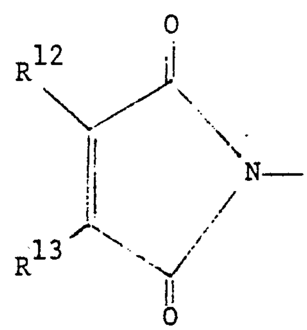

J-2

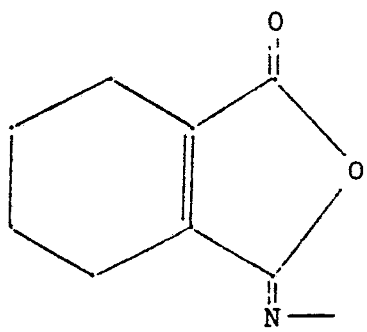

J-3

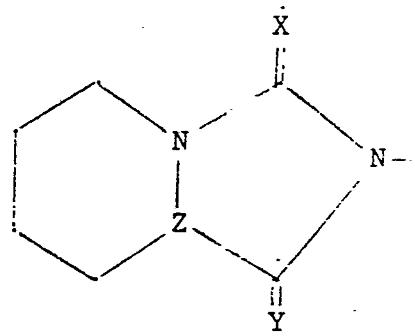

J-4

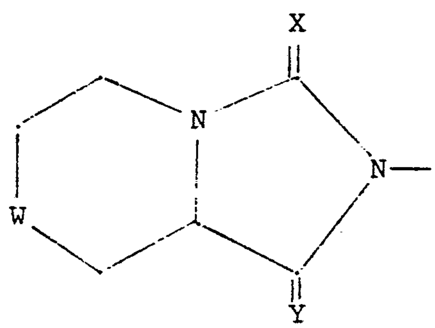

J-5

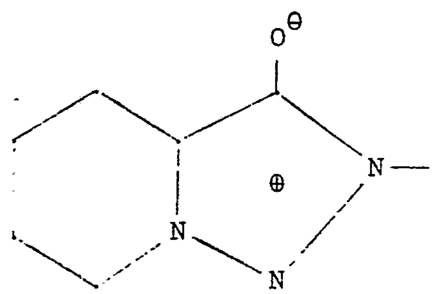

J-6

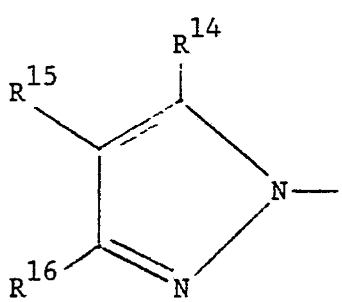

J-7

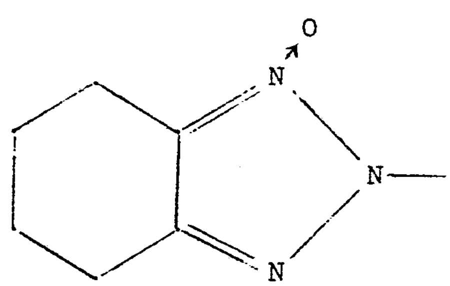

J-8

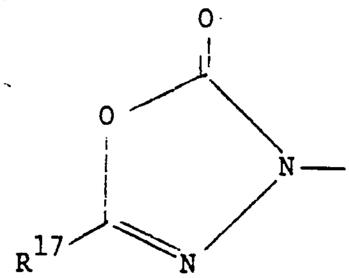

J-9

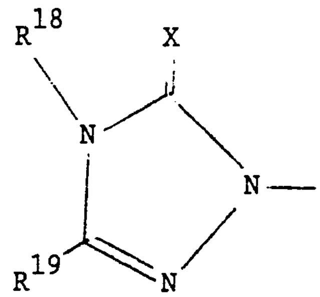

J-10

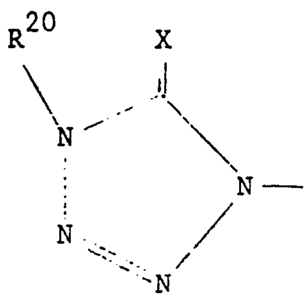

J-11

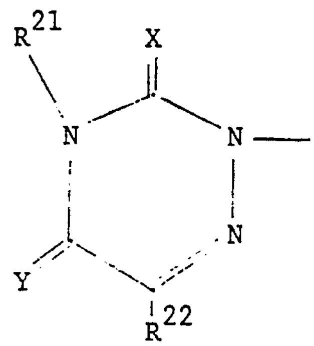

J-12

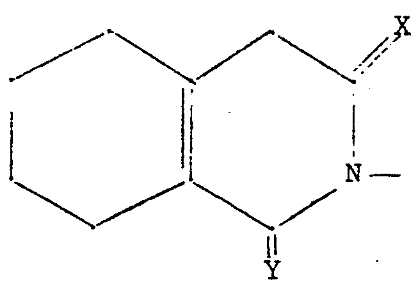

J-13

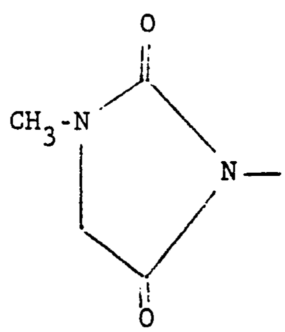

J-14

wherein

X and Y each is O or S;

Z is C or N;

W is S or $SO_2$;

m is zero, one or two;

$R^{11}$ is a halogen atom, or is an alkyl, haloalkyl or alkoxy group having one to four carbon atoms:

$R^{12}$ and $R^{13}$ each independently represents a hydrogen atom or an alkyl group having one to four carbon atoms, or $R^{12}$ and $R^{13}$ together with the carbon atoms to which they are attached form an alicyclic six-membered ring that may be substituted by one to three methyl groups;

$R^{14}$ is a hydrogen or halogen atom; or an alkyl, or alkyl-$S(O)_a$- group having one to four carbon atoms wherein a is zero, one or two;

$R^{15}$ is a hydrogen or halogen atom; or an alkyl, alkylthio or alkylsulfonyl group having one to four carbon atoms;

$R^{16}$ is an alkyl group having one to four carbon atoms, or $R^{15}$ and $R^{16}$ together form the moiety, $-(CH_2)_b-$, wherein b is three or four, which may be substituted by one to three methyl groups;

$R^{17}$ is a hydrogen atom or an alkyl, alkenyl, haloalkyl or alkoxyalkyl group having up to six carbon atoms, or a cycloalkyl group having three to six carbon atoms, a cycloalkylalkyl group having four to eight carbon atoms, or either of these groups substituted by one to three alkyl groups each having one to four carbon atoms;

$R^{18}$ has one to six carbon atoms and is an alkyl, haloalkyl, cyanoalkyl, alkoxyalkyl, alkenyl, alkynyl, or -alkyl-$S(O)_a$-alkyl group where a is zero, one or two;

$R^{19}$ is a hydrogen atom, a group $R^{18}$ as defined above, or $R^{18}$ and $R^{19}$ together represent an alkylene moiety $-(CH_2)_b-$, as defined above for $R^{16}$, or a moiety $-(CH_2)_c-U-(CH_2)_d-$ wherein c and d each is zero, one, two or three, and c and d = two or three, and U is oxygen, $-S(O)_a-$ or -NR alkyl- where a is zero,

one or two, with the proviso that when U is $-S(O)_a-$, c is other than zero;

$R^{20}$ has one to six carbon atoms, and is an alkyl, haloalkyl, alkoxyalkyl, alkylthioalkyl, cyanoalkyl, haloalkoxyalkyl, alkenyl and haloalkenyl group;

$R^{21}$ is a hydrogen atom, or has one to four carbon atoms and is an alkyl, haloalkyl, cyanoalkyl, alkenyl, alkynyl, alkoxyalkyl, amino, or alkoxycarbonyl group;

$R^{22}$ is a group $R^{20}$ as defined above, or is a carboxyl or alkoxycarbonyl group;

or J is benzhydrylamino, benzoylamino, acetylamino, benzylamino, allylamino, trichloroacetylamino, amino or isocyanato;

and salts of the acidic species with alkali metal bases, ammonia and amines.

In these compounds, each aliphatic group can be either straight chain or branched-chain. By "halogen" is meant bromine, chlorine and fluorine. Suitable amine salts are those of mono-, di- and tri-alkyl- and -alkanol- amines wherein each alkyl moiety contains from one to twenty carbon atoms. The heteroaryl and heteroaralkyl moieties suitably are those wherein the heteroaryl moiety is furanyl, pyrrolyl, thienyl, pyridinyl, thiazolyl, isoxazolyl, pyrazolyl, imidazolyl, triazolyl (1,2,3- or 1,2,4-), benzofuranyl, indolyl, indazolyl, thianaphthenyl, pyridazinyl, pyrimidinyl, pyrazinyl, cinnolinyl, quinazolinyl or quinoxalinyl.

Of particular interest are compounds of the formula IA, particularly those wherein $R^1$ is a halogen atom, such as chlorine, and J is one of J-1 and J-2, wherein m is zero and $R^3$ and $R^4$ together with the carbon atoms to which they are attached form an alicyclic six-membered ring.

Compounds of Formula I wherein J is J-1 or J-2, X and Y each is an oxygen atom, $R^7$ is $-CH(R^2)(R^3)$, and $R^6$ is a hydrogen atom, can be prepared by cyclizing appropriate J-1 and J-2 phenols of the formulae:

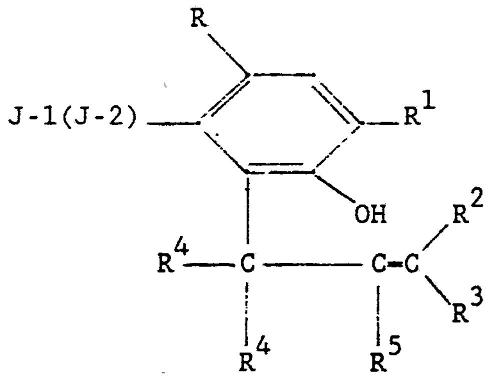

(A)

and

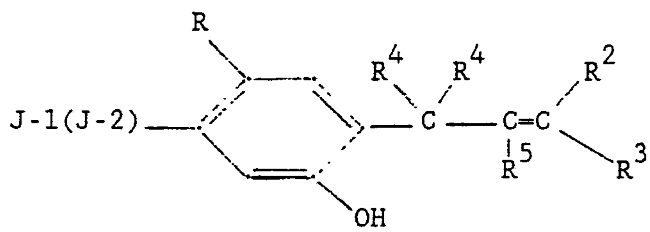

(B)

(II)

The cyclization can be effected by one or more of three general methods:

(a) thermal: the phenol precursor II, optionally in a solvent such as xylene, is heated at 150-250°C;

(b) acid catalyzed: refluxing a solution of the phenol precursor 1I in an inert solvent, such as xylene, containing a catalytic amount of p-toluenesulfonic acid (PTSA).

(c) free-radical: treating the phenol precursor II with hydrogen bromide in the presence of a peroxide.

Such methods are described by L.I. Smith, Chemical Reviews, Page 287, et seq., 1940.

Compounds of Formula I wherein J is J-1 or J-2 also can be prepared by treating a phenol II with a moderate stoichiometric excess of an epoxidizing agent such as peracetic acid, m-chloroperoxy-benzoic acid or like reagent, in an inert solvent

such as methylene chloride at a temperature of about 0-80°C, to form the epoxides

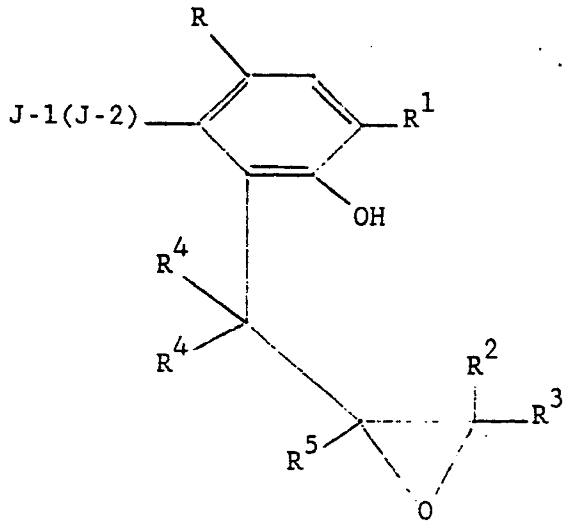

and

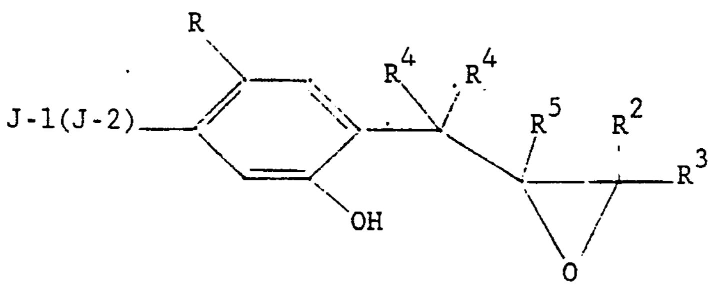

(III)

The reaction preferably is conducted by refluxing the mixture of phenol, epoxidizing agent and solvent until the reaction is complete, then treating the mixture with an aqueous solution of sodium sulfite until no reaction is obtained on starch/potassium iodide paper.

The epoxide may spontaneously cyclize at room temperature, or it may be cyclized by (a) an acid catalyst (PTSA) and/or like protic catalyst in an inert solvent such as 1,2-dichloroethane, or (b) a basic catalyst, such as sodium hydride or a tertiary amine, in an inert solvent such as tetrahydrofuran (THF) or

N,N-dimethylformamide (DMF) at a temperature of about $0^{o}C$ to $100^{o}C$. The products have the formulae

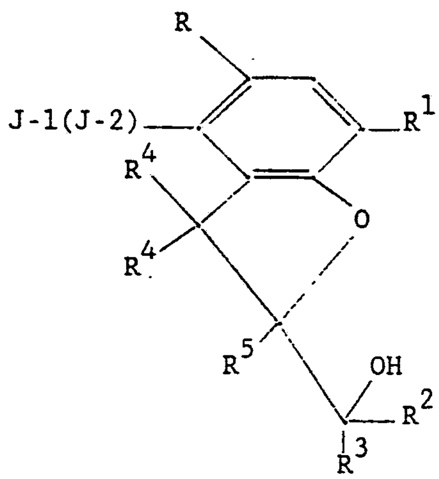

(A)

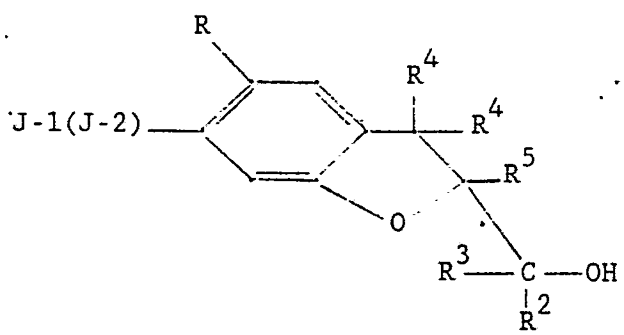

(B)

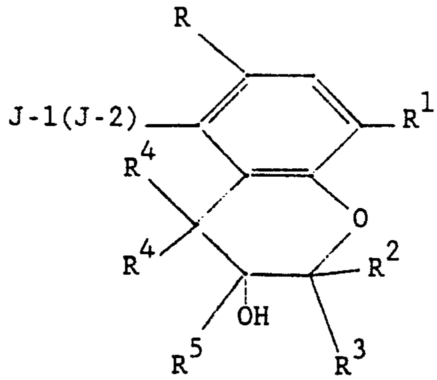

(C)

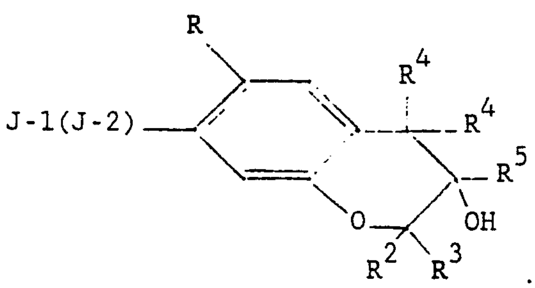

(D) (IV)

The phenol precursors II can be prepared by Claisen rearrangement of the appropriate J-1 or J-2 compounds of the formula

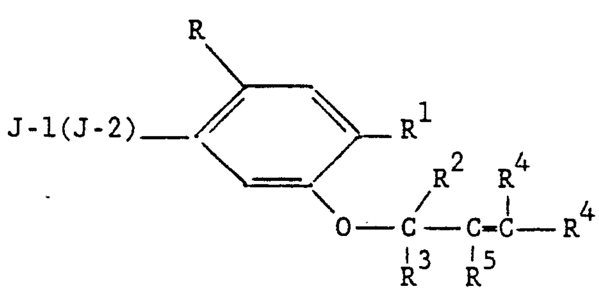

(V)

When $R^1$ is hydrogen, the product is ordinarily a mixture of the two isomeric forms IIA and IIB. When $R^1$ is other than hydrogen, only the isomeric form IIA is obtained. The rearrangement is effected by heating the allyloxyphenyl precursor V at a moderately elevated temperature - for example a temperature within the range of from about 150°C to about 250°C. The precursor V per se (i.e., neat) may be used or it may be in solution in a suitable inert solvent. Alternatively, the rearrangement can be effected at about 0°C to room temperature by employing a Lewis acid catalyst, such as boron trichloride, in an inert solvent, such as methylene chloride. It is to be noted that in some cases, at least, when the precursor is heated at a temperature within the upper portion of the indicated range - for example, above about 200°C - cyclization may occur, in addition to the rearrangement.

Generally speaking, mixtures of the benzofuran and benzopyran will be obtained from all the cyclization procedures, the relative proportions of the two materials depending upon the character of the moieties $R^2$ and $R^3$ and the method, (a), (b), (c), or epoxidation, followed by treatment with acid or base to effect the cyclization.

Allyloxphenyl precursors V can be prepared by treating a solution of the appropriates J-1 or J-2 compounds of the formula

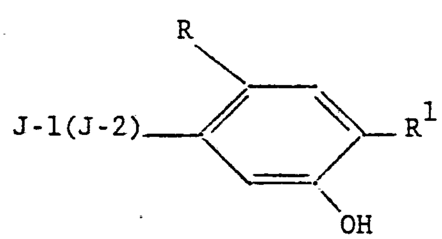

(VI)

with appropriate allyl halides,

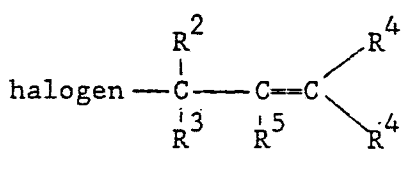

(VII)

preferably the bromide, by refluxing a solution of the halide in an inert solvent in the presence of potassium carbonate.

Precursor phenols VI can be prepared by conventional procedures, as by heating a mixture of an appropriate phthalic, 3,4,5,6-tetrahydrophthalic, quinolinic, or maleic anhydride, and an appropriate aniline

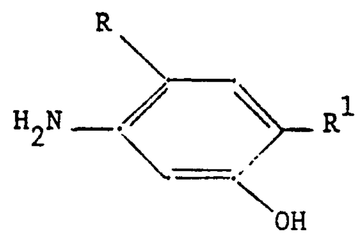

(VIII)

in acetic acid. Procedures for preparing such precursor phenols VI are well known, being described inter alia, in U.S. patent 4,452,981, European Patent Application 61,741 and United Kingdom Patent application 2,046,754, and in references cited therein.

Alternatively, allyloxyphenyl precursors V can be prepared by heating a mixture of an appropriate anhydride, such as phthalic, 3,4,5,6-tetrahydrophthalic, quinolinic, or maleic anhydride, and an appropriate allyloxyaniline of the formula

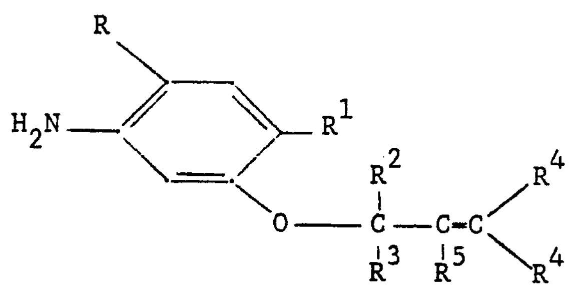

(IX)

in acetic acid, these anilines being prepared from the corresponding allyloxynitrobenzenes (as by treating the nitrobenzene with stannous chloride in a solvent such as ethyl acetate), the allyloxynitrobenzenes being prepared by treating the corresponding nitrophenols with the appropriate allyl halides VII.

Compounds of formulae IA and IB, wherein $R^7$ is one of the defined functional moieties can be prepared by treatment of an appropriate compound of Formula I by conventional procedures and reagents, as illustrated in the Examples, hereinafter. Thus; various subclasses of compounds of formulae IA and IB can be prepared as follows from precursors wherein $R^7$ is $-CH(R^9)-O(R^{10})$ and $R^{10}$ is hydrogen:

Compounds wherein $R^7$ is $-CH(R^9)-O(R^{10})$ wherein $R^{10}$ is other than hydrogen, can be prepared (1) by treatment of an alkali metal (preferably sodium) derivative of an appropriate compound of Formula I wherein $R^7$ is $-CH(R^9)-OH$ with an appropriate halide $R^{10}$-halogen or sulfate $(R^{10})_2SO_4$ to produce an ether; (2) by treatment of the alcohol $-CH(R^9)-OH$ with a carboxylic acid anhydride or acid chloride to produce as ester; (3) by treatment of the alcohol with an alkyl- or phenyl-sulfonyl chloride to produce a sulfonate; or (4) by treatment of the alcohol with cyanic acid, an alkyl cyanate, or a carbamoyl chloride to produce carbamates.

Compounds wherein $R^7$ is $-C(R^9)=NO(R^8)$ also are prepared by conventional procedures; oxidation of an appropriate compound of Formula IA and IB wherein $R^7$ is $-CH(R^9)OH$ with a mixture of oxalyl chloride, dimethyl sulfoxide and triethylamine to form an aldehyde or ketone wherein $R^7$ is $-C(O)-(R^9)$, which can be treated with a hydroxylamine to form the oxime, $(-C(R^9)=NOR^8)$.

Compounds wherein $R^7$ is $-C(O)-O(R^8)$, $-C(O)N(R^8(R^8)$, $-C(O)Cl$ or -CN can be prepared by conventional treatment of an alcohol, $-CH(R^9)-O(R^{10})$ and $R^9$ and $R^{10}$ are both hydrogen, with Jones' reagent or pyridinium dichromate to give the corresponding carboxylic acid, $R^7$ is $-C(O)OH$, which can be converted to a salt, or to an $R^8$ ester, or to an amide $-C(O)N(R^8)(R^8)$, by treatment of the acid with thionyl chloride to form the acid chloride ($R^7$ = $-C(O)Cl$) and treatment of the acid chloride with an appropriate alcohol $R^8$-OH or amine $H-N(R^8)(R^8)$, respectively. Treatment of the acid chloride with ammonia produces the

unsubstituted amide, which is dehydrated by conventional procedures to the nitrile, $R^7$ = -CN.

Compounds wherein $R^7$ is $-CH(R^9)CN$ are prepared by treating an appropriate compound of Formula IA and IB wherein $R^7$ is $-CH(R^9)OH$, with mesyl chloride to form the mesylate, and treating the mesylate with potassium cyanide.

Compounds wherein $R^7$ is $-CH(R^2)(R^3)$ and $R^3$ is halogen, are prepared by treating an appropriate compound of Formula I wherein $R^7$ is $-CH(R^2)OH$ with a carbon tetrahalide in the presence of triphenylphosphine.

Compounds of formulae IC and ID, wherein $R^6$ is one of the defined functional moieties, can be prepared by treating an appropriate compound of Formula I wherein $R^5$ is hydrogen and $R^6$ is hydroxyl by conventional procedures and reagents, as described above for the moiety $R^7$. Thus, when $R^5$ is hydrogen and $R^6$ is -OH, the compound of Formula IC or ID may be oxidized to a ketone, which can be converted to an oxime, or converted to the mesylate, which can be used as precursor for the preparation of a nitrile, carboxylic acid, acid chloride, ester or amide.

Compounds of subclasses IC and ID wherein $R^5$ is hydrogen and $R^6$ is hydroxyl can be converted to those wherein $R^5$ and $R^6$ together represent an oxo (=O) oxygen atom by treating the compound wherein $R^5$ is hydrogen and $R^6$ is hydroxyl with Jones' reagent.

Compounds of Formula I can be prepared by the following general methods:

J = J-1, J-2, (Z = carbon or $R^{12}$ and $R^{13}$ complete a ring): in addition to the methods already described, these compounds can be prepared by treating an appropriate phthalic, tetrahydrophthalic or maleic anhydride with an appropriate aniline, by fusing the two reagents at a temperature of 120-220°C, or by refluxing a mixture of the reagents in a solvent such as acetic acid, toluene or xylene. The reaction can be catalyzed in xylene or toluene by an amine such as triethylamine or DBU (1,8-diazabicyclo[5.4.0]undec-7-ene). The

products wherein one or both of X and Y is (are) sulfur can be prepared by treating (i.e., thionating) a product wherein X and Y are both oxygen with phosphorus pentasulfide or Lawesson's Reagent (2,4-bis-(4-methoxyphenyl)-1-,3-dithia-2,4-diphosphetane-2, 4-disulfide), in an inert solvent such as benzene, toluene, xylene or chloroform at room temperature to reflux temperature. Where Z = nitrogen, an appropriate quinolinic anhydride is treated with an appropriate aniline in two steps: (a) stirring the reagents in an inert solvent such as methylene chloride or tetrahydrofuran at room temperature to reflux temperature, to give the intermediate amide/acid, then (b) dehydrating the intermediate with acetic anhydride at 80°C to reflux temperature. Alternatively the two reagents are stirred at room temperature in toluene to form the intermediate, which then is treated with 0.1 to 0.5 equivalent of triethylamine as catalyst, followed by azeotropic distillation to yield the product. References: U.S. patents 4,406,690; 4,439,229; U.K. patent 2,150,929;

J = J-3: such are prepared by the method described above for preparing the J-1 and J-2 compounds wherein Z = nitrogen, except that the amide/acid intermediate is dehydrated by using DCC (1,3-dicyclohexylcarbodiimide) or like dehydrating/condensation agent. References: U.S. patent 4,472,190.

J = J-4, Z = nitrogen, X = O, Y = O or S: 1-(ethoxycarbonyl)piperidazine is treated with an iso(thio)cyanate prepared from an appropriate aniline by conventional methods, and treating the resulting intermediate product with sodium hydride. Alternatively, an appropriate N-(alkoxycarbonyl)hydrazine is treated with an appropriate iso(thio)cyanate, and the resulting intermediate is cyclized with a base to provide a urazole. Alkylation of the disodium salt of the urazole with 1,4-dibromobutane yields the desired product. Reference: European patent 104484; German Offenlegungschrift 3,504,051. J-4 compounds wherein Z = carbon, X = O or S, Y = O: ethyl pipecolinate is treated with the iso(thio)cyanate prepared from

the appropriate aniline, in an inert solvent, such as THF, and the resulting (thio)urea intermediate is treated with ethanolic hydrogen chloride at reflux. Reference: U.S. Patent 4,560,752. Thionation as described for J = J-1, provides those compounds wherein both X and Y = S.

J = J-5; these are prepared by methods analogous for those described for J = J-4, Z = nitrogen except that the (thio)urea intermediate is treated with either aqueous hydrochloric acid or sodium methoxide in methanol. Reference: European Patent 70,389.

J = J-6; an appropriate aniline is diazotized and coupled with pipecolinic acid in the presence of triethylamine. Reference: U.S. patents 4,599,104; 4,002,636; 3,939,174.

J = J-7: an appropriate aniline is diazotized, and the product is reduced with stannous chloride to give a hydrazine. The hydrazine is treated with a 2-(alkoxycarbonyl)cyclohexanone under dehydrating conditions to yield the hexahydroindazol-3-one, which is chlorinated, optionally in the presence of a dehydrohalogenating agent to provide the J-7 compound wherein $R^{14}$ is halogen. Reference: European patent 152,890. Preparation of such compounds, and those wherein $R^{14}$ is other than halogen is described in European patent 138,527. These patents also teach methods for preparing J-7 compounds wherein $R^{15}$ and $R^{16}$ differ from species to species.

J = J-8: an appropriate aniline is converted to a hydrazine, which is treated with 6-(hydroxyimino)-1-morpholinocyclohexane, and the resulting product is oxidized with cuprous sulfide in pyridine. Reference: European patent 142,769.

J = J-9: an appropriate aniline is converted to a hydrazine, which is treated with an acyl halide to form the acylhydrazide, which is treated with trichloromethyl chloroformate or phosgene, optionally in the presence of triethylamine.

J = J-10: an appropriate aniline is converted to a hydrazine, which is condensed with an alpha-ketoacid to form a hydrazone. The hydrazone is treated with diphenylphosphoryl azide in the presence of triethylamine to give a J-10 compound wherein X = O, $R^{18}$ = hydrogen, which can be thionated to X = S with phosphorus pentasulfide. Such compounds can be alkylated by conventional procedures to form those wherein $R^{18}$ = alkyl. References: U.S. patents 4,213,773; 4,315,767; WIPO patent WO 85/01637.

J = J-11: an iso(thio)cyanate prepared from the appropriate aniline is treated with trimethylsilyl azide ($R^{20}$ = hydrogen), which may be alkylated by conventional means. Reference: WIPO patent WO 85/01939.

J = J-12: an appropriate aniline is diazotized and treated with malonyldiurethane. The resulting product is cyclized by treatment first with ethanolic potassium hydroxide in THF, then with aqueous hydrochloric acid to give a triazinedionecarboxylic acid, which is decarboxylated in the presence of mercaptoacetic acid and xylene to give J-12 wherein $R^{21}$ = hydrogen, which may be converted to $R^{21}$ = one of the defined moieties other than hydrogen. Alternatively, an appropriately substituted aniline is treated with sodium nitrite and tin (II) chloride in aqueous hydrochloric acid to produce the corresponding hydrazine, which is converted to the hydrazone by treatment with acetone in sulfuric acid and THF. Treatment of the hydrazone with potassium cyanate in aqueous acetic acid gives a triazolidinone, which upon treatment with $R^{22}COCO_2H$ and sulfuric acid in dioxane gives J-12 wherein $R^{21}$ = hydrogen. Reference: WIPO patent WO 86/00072. The patent also describes other suitable methods.

J = J-13: these are prepared by treating tetrahydrohomophthalic acid (2-carboxycyclohexene-1-acetic acid: (R. Grewe and A. Mondon, Chemische Berichte, volume 81, pages 279-286 (1948)), at page 283) with an appropriate aniline in refluxing acetic acid solvent.

J = J-14: an isocyanate prepared from an appropriate aniline is treated with sarcosine methyl ester hydrochloride in the presence of triethylamine, followed by treatment of the resulting urea with aqueous ethanolic hydrogen chloride. Reference: U.S. patent 4,560,752.

An appropriate aniline or isocyanate reagent can be prepared from a compound of Formulae I, or a compound of Formulae I wherein J is other than one of the groups defined herein, prepared by methods described herein. In such a case, the moiety J might appropriately be one of benzhydrylamino, benzoylamino or acetylamino, benzylamino, allylamino and trichloroacetylamino. As is shown in the examples, the aniline or isocyanate can be prepared from the J-compound by conventional means.

It is to be understood that the following classes of compounds are novel and constitute aspects of this invention:

(1) Compounds of Formula I, including those wherein J is one of J--1 through J-14, those wherein J is one of the moieties described in the paragraph immediately above, and those wherein J is amino ($-NH_2$) or isocyanato (-NCO);

(2) Compounds of Formula II;

(3) Compounds of Formula III;

(4) Compounds of Formula IV;

(5) Compounds of Formula V;

(6) Compounds of Formula VI;

(7) Compounds of Formula IX.

The preparation, isolation and testing of typical individual species of the compounds of Formulae I are described in the examples following. The illustrated compounds are of formula IA wherein $R^4$ is hydrogen, and in the interest of brevity, and clarity, and to avoid repetition of sometimes long chemical names, these species will be identified in terms of Formula IA and the other symbols used therein.

| Compound | R | $R^1$ | $R^5$ | $R^7$ |
|---|---|---|---|---|
| A | H | Cl | methyl | methyl |
| B | F | Cl | methyl | methyl |
| C | F | methyl | H | methyl |
| D | H | Br | H | methyl |
| E | F | Cl | H | ethyl |
| F | F | Cl | H | isopropyl |
| G | Cl | Cl | methyl | methyl |

The following compounds are examples of those of formula IA wherein $R^4$ and $R^5$ are hydrogen and $R^7$ is $-CH_2-O-R^{10}$

| Compound | R | $R^1$ | $R^{10}$ |
|---|---|---|---|
| H | H | Cl | phenyl-C(O)- |
| I | H | Cl | isopropyl |
| J | H | Cl | tert-butyl-C(O)- |
| K | H | Cl | methylcyclopropyl-C(O)- |
| L | H | Cl | methyl-NH- |
| M | H | Cl | 4-chlorophenyl-$S(O)_2$- |
| N | F | Cl | ethyl |
| O | F | Cl | t-butyl |
| P | F | Cl | methylcyclopropyl-C(O)- |
| Q | F | Cl | methyl-NH-C(O)- |
| R | H | Br | methyl-$SO_2$- |
| S | H | Br | t-butyl |
| T | H | Br | methylcyclopropyl-C(O)- |
| U | H | methyl | isopropyl |
| V | F | methyl | methyl |
| W | Cl | Cl | methyyl-C(O)- |
| X | Cl | Cl | isopropyl |
| Y | H | Cl | $(methyl)_2$N-C(O)- |
| Z | H | Cl | (methyl)(methoxy)N-C(O)- |
| AA | H | Cl | 4-chlorophenyl-C(O)- |

The following compounds are examples of those of formula IA wherein $R^5$ is hydrogen and $R^7$ is $-CH=N-O-R^8$.

| Compound | R | $R^1$ | $R^8$ |
|---|---|---|---|
| AB | H | Cl | isopropyl |
| AC | H | Cl | $-CH_2C(O)O$-ethyl |
| AD | H | Cl | $-CH(CH_3)C(O)O$-ethyl |
| AE | H | Cl | allyl |
| AF | H | Cl | methallyl |
| AG | F | Cl | isopropyl |
| AH | H | Br | isopropyl |
| AI | H | methyl | isopropyl |
| AJ | F | methyl | isopropyl |
| AK | Cl | Cl | isopropyl |
| AL | F | Cl | $-CH_2C(O)O$-ethyl |

The following compounds are examples of those of formula IA wherein $R^5$ is hydrogen and $R^7$ is $-C(O)OR^8$.

| Compound | R | $R^1$ | $R^8$ |
|---|---|---|---|
| AM | H | Cl | allyl |
| AN | H | Cl | methallyl |
| AO | H | Cl | propargyl |
| AP | H | Cl | $-CH(CH_3)C(O)OC_2H_5$ |
| AQ | F | Cl | allyl |
| AR | F | methyl | allyl |
| AS | Cl | Cl | allyl |
| AT | H | methyl | allyl |
| AU | F | Cl | propargyl |
| AV | F | methyl | $-CH(CH_3)C(O)OC_2H_5$ |
| AW | F | methyl | propargyl |

In all cases, those compounds wherein J = J-1, m = 0, Z = C and those wherein J = J-2, $R^{12} + R^{13} = -(CH_2)_4-$, are particularly preferred.

Compounds of Formula I have been found to affect adversely the growth of some plants, many of which are commonly considered as weeds, and therefore are useful for controlling the growth of such unwanted plants. Compounds of Formula I have been found to have selectivity with respect to some crop plants - i.e., they control weeds at dosages at which they do not significantly harm

the crop plants. While compounds of Formula I are active when applied pre-emergence or preplant incorporated (applied to the soil before the seeds have sprouted), most appear to be more effective when applied postemergence (applied to the foliage of the growing plant).

Accordingly, the invention includes a method combating unwanted plants which comprises applying to the locus an effective amount of a compound of Formula I. In the cases where it is desired to control weeds in crop plantings, it is of course preferable to employ the lowest dosage that will control the weeds, for this will minimise any possible deleterious effect of the compound upon the crop plants.

For application, a compound of Formula I ordinarily is applied most effectively by formulating it with a suitable inert carrier or surface-active agent, or both. The invention, therefore, also includes compositions suitable for combating unwanted plants, such compositions comprising an inert carrier or surface-active agent or both, and as active ingredient at least one compound of Formula I.

The term "carrier" as used herein means an inert solid or liquid material, which may be inorganic or organic and of synthetic or natural origin, with which the active compound is mixed or formulated to facilitate its application to the plant, seed, soil or other object to be treated, or its storage, transport and/or handling. Any of the materials customarily employed in formulating pesticides, herbicides, or fungicides - i.e., horticulturally acceptable carriers - are suitable.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements such as, for example, carbon and sulphur;

natural and synthetic resins such as, for example, coumarone resins, polyvinyl chloride, and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Example of suitable liquid carriers are water, alcohols such as isopropyl alcohol and glycols; ketones such as acetone, methyl ethyl ketone, methyl isobutyl ketone and cyclohexanone; ethers such as cellosolves; aromatic hydrocarbons such as benzene, toluene and xylene; petroleum fractions such as kerosene and light mineral oils; chlorinated hydrocarbons such as carbon tetrachloride, perchloroethylene and trichloromethane. Also suitable are liquefied, normally vapourous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products; alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene

sulfonate; and polymers of ethylene oxide and copolymers of ethylene oxide and propylene oxides.

The compositions of the invention may be prepared as wettable powders, dusts, granules, solutions, emulsifiable concentrates, emulsions, suspension concentrates and aerosols. Wettable powders are usually compounded to contain 25 to 75% by weight of active compound and usually contain, in addition to the solid carrier, 3-10% by weight of a dispersing agent, 2-15% of a surface-active agent and, where necessary, 0-10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0.5-10% by weight of the active compound. Granules are usually prepared to have a size between 10 and 100 BS mesh (1.676-0.152 mm), and may be manufactured by agglomeration or impregnation techniques. Generally, granules will contain 0.5-25% by weight of the active compound, 0-1% by weight of additives such as stabilizers, slow release modifiers and binding agents. Emulsifiable concentrates usually contain, in addition to the solvent and, when necessary, cosolvent, 10-50% weight per volume of the active compound, 2-20% weight per volume emulsifiers and 0-20% weight per volume of appropriate additives such as stabilizers, penetrants and corrosion inhibitors. Suspension concentrates are compounded so as to obtain a stable, non-sedimenting, flowable product and usually contain 10-75% weight of the active compound, 0.5-5% weight of dispersing agents, 1-5% of surface-active agent, 0.1-10% weight of suspending agents, such as defoamers, corrosion inhibitors, stabilizers, penetrants and stickers, and as carrier, water or an organic liquid in which the active compound is substantially insoluble; certain organic solids or inorganic salts may be dissolved in the carrier to assist in preventing sedimentation or as antifreeze agents for water.

Of particular interest in current practice are water-dispersible granular formulations. There are in the form of dry, hard granules that are essentially dust-free, and are resistant to attrition on handling, thus minimizing the formation of dust. On contact with water, the granules readily disintegrate to form stable suspensions of the particles of active material. Such formulations contain 90% or (up to 95%) more by weight of finely divided active material, 3-7% by weight of a blend of surfactants, which act as wetting, dispersing, suspending and binding agents, and may contain up to 3% by weight of a finely divided carrier, which acts as a resuspending agent.

Aqueous dispersions and emulsions, for example, compositions obtained by diluting a wettable powder or a concentrate according to the invention with water, also lie within the scope of the present invention. The said emulsions may be of the water-in-oil or of the oil-in-water type, and may have thick, mayonnaise-like consistency.

It is evident from the foregoing that this invention contemplates compositions containing as little as about 0.5% by weight to as much as about 95% by weight of a compound of Formula I as the active ingredient.

The compositions of the invention may also contain other ingredients, for example, other compounds possessing pesticidal, especially insecticidal, acaricidal, herbicidal or fungicidal properties, as are appropriate to the intended purpose.

Protection of a locus or area from undesirable plants is effected by applying a compound of Formula I, ordinarily in a composition of one of the aforementioned types, to soil in which the seeds of the unwanted plants are present, or to the foliage of the unwanted plants. The active compound, of course, is applied in an amount sufficient to exert the desired action.

The amount of the compound of the invention to be used in combating undesired plants will naturally depend on the condition of the plants, the degree of activity desired, the

formulation used, the mode of application, the climate, the season of the year, and other variables. Recommendations as to precise amounts are, therefore, not possible. In general, however, application to the locus to be protected of from 0.02 to 10.0kg per hectare of the compound of Formula I will be satisfactory.

The following examples describe the preparation, isolation and physical properties of typical individual compounds of Formulae I, in particular instances. In each case, the identity of each product, and each of any intermediate involved, was confirmed by appropriate chemical and spectral analyses.

Example 1

N-(2,3-dihydro-2-methyl-6-benzofuranyl)-3,4 5,6-tetrahydrophthalimide (1)

A mixture of 25.0 g of 3,4,5,6-tetrahydrophthalic anhydride (THPA), 17.9 g of 3-aminophenol and 350 ml of glacial acetic acid was refluxed for 1 hour, then cooled and poured slowly into 1400 ml of ice-water. The resulting mixture was filtered. The collected solids were washed with cold water and dried (oven, reduced pressure) to give N-(3-hydroxyphenyl)-3,4,5,6-tetrahydrophthalimide (1A), as yellow crystals, m.p.: 196-197.5°C.

3.0 g of powdered potassium carbonate, then 2.6 g of allyl bromide were added to a stirred suspension of 5.0g of 1A in 25 ml of acetone. The mixture was refluxed for 1 hour, then stirred overnight at room temperature. The solvent was evaporated, water was added to the residue and the pH of the mixture was adjusted to 11 by addition of 10% aqueous sodium hydroxide. The resulting mixture was extracted with ethyl acetate, the extract was washed with brine and dried ($Na_2SO_4$), the solvent was evaporated and the residue was flash chromatographed on silica gel with a 1:9 v:v mixture of ethyl acetate and hexane as eluent to give N-(3-allyloxyphenyl)-3,4,5,6-tetrahydrophthalimide (1B), as yellow crystals, m.p.: 88-90°C.

1 g of 1B was heated on an oil bath at 200-210°C for 5 hours and the product was flash chromatographed on silica gel with a 1:4 v:v mixture of ethyl acetate and hexane as eluent. Two products were isolated, N-(4-allyl-3-hydroxyphenyl)-3,4,5,6-tetrahydrophthalimide (IC), as yellow crystals, m.p.: 147-148°C and N-(2-allyl-3-hydroxyphenyl)-3,4,5,6-tetrahydrophthalimide (ID), as a white solid, mp.: 72-75°C.

A mixture of 0.20 g of IC, 100 mg of p-toluenesulfonic acid (PTSA), and 10 ml of xylene was refluxed for 24 hours, then cooled and poured into a saturated aqueous solution of sodium bicarbonate. The resulting mixture was extracted with ethyl

acetate, and the extract was washed in succession with sodium bicarbonate solution, water and brine, dried ($Na_2SO_4$) and filtered. The solvent was evaporated and the residue was chromatographed with a Chromatotron plate, starting with a 3:17 v:v mixture of ethyl acetate and hexane as eluent and gradually increasing the ethyl acetate content in the eluent, to give 1, as yellow crystals, m.p.: 114-118°C.

Example 2

N-(2,3-dihydro-2-methyl-4-benzofuranyl)-3,4,5,6-tetrahydrophthalimide (2)

2 was prepared, as a yellow oil, by treating 0.48 g of 1D according to the procedures described in Example 1 for preparing 1 from 1C.

Example 3

N-(7-chloro-2,3-dihydro-2-methyl-4-benzofuranyl)-3,4,5,6-tetrahydrophthalimide (3)

A mixture of 7.4 g of 2-chloro-5-nitrophenol, 5.4 g of allyl bromide, 5.9 g of anhydrous potassium carbonate and 50 ml of dry N,N-dimethylformamide (DMF) was stirred at room temperature over a weekend. Then the mixture was poured into water, and the resulting mixture was extracted twice with ethyl acetate and twice with ether. The combined extracts were washed with water, then with brine, dried ($Na_2SO_4$), filtered and stripped of the solvents. The residue was recrystallized from hexane to give 1-(allyloxy)-2-chloro-5-nitro-benzene (3A), as orange needles, m.p.: 58-59.5°C.

A suspension of 1.0 g of 3A and 5.3 g of stannous chloride dihydrate in 10ml of absolute ethanol was heated for 1 hour. The resulting mixture was poured into 100 g of ice, then sufficient 10% aqueous sodium hydroxide solution was added, with stirring, to bring the pH of the mixture to 10. The mixture was extracted with ethyl acetate, then with ether. The combined extracts were washed with water, then brine, then dried ($Na_2SO_4$) and stripped of the solvents, to give 3-(allyloxy)-4- chloroaniline (3B), as a brown oil.

A mixture of 4.4 g of THPA, 5.3 g of 3B and 60 ml of glacial acetic acid was refluxed for 1 hour, then poured into 300 ml of ice water. The resulting mixture was allowed to stand for 10 minutes, then filtered. The collected solid was washed with cold water, dried under reduced pressure and recrystallized from ethyl acetate/hexane, to give N-(3-(allyloxy)-4-chlorophenyl)-3, 4,5,6-tetrahydrophthalimide (3C), as yellow crystals, m.p.: 118.5-120°C.

7.2g of 3C was heated at 210°C for 1.5 hours, then the product was cooled and flash chromatographed on silica gel, with a 1:5 v:v mixture of ethyl acetate and hexane as eluent, to yield a viscous yellow oil that solidified on standing to give N-(2-allyl-4-chloro-3-hydroxyphenyl)-3,4,5,6-tetrahydrophthalimide (3D), m.p.: 80-82.5°C.

3 was prepared, as yellow crystals, m.p.: 134-137°C, from 3D by the procedures described in Example 1 for preparing 1 from 1C.

Example 4

N-(7-chloro-2,3-dihydro-2-(hydroxymethyl)-4-benzofuanyl)-3,4,5,6-tetrahydrophthalimide (4)

A solution of 0.37 g of m-chloroperbenzoic acid (MCPBA) in 5 ml of methylene chloride was added dropwise to a solution of 0.5 g of 3D in 10 ml of methylene chloride at room temperature. The resulting mixture was refluxed overnight, cooled and stirred with a few millilitres of saturated aqueous sodium sulfite solution, until no reaction to starch/iodide paper occurred. The organic phase was separated, washed with saturated aqueous sodium bicarbonate solution, dried ($Na_2SO_4$) and stripped of solvent. The residue was flash chromatographed over silica gel, with a 2:3 v:v mixture of ethyl acetate and hexane as eluent, to obtain 4, as yellow crystals, m.p.: 196-197.5°C.

Example 5

N-(2,3-dihydro-2,7-dimethyl-4-benzofuranyl)-3,4,5,6-tetrahydrophthalimide (5)

N-(2-allyl-3-hydroxy-4-methylphenyl)-3,4,5,6-tetrahydrophthalimide (5A) was prepared, as yellow crystals, m.p.: 141-143°C, from 2-methyl-5-nitrophenol and allyl bromide by the procedures described in Example 3 for preparing 3D from 2-chloro-5-nitrophenol and allyl bromide.

5 was prepared, as yellow crystals, m.p.: 158-160°C, from 5A, according to the procedure described in Example 1 for preparing 1 from 1C.

Example 6

N-(2,3-dihydro-2(hydroxymethyl)-7-methyl-4-benzofuranyl)-3,4,5,6-tetrahydrophthalimide (6)

6 was prepared, as yellow crystals, m.p.: 177-179°C, from 5A, by the procedures described in Example 4 for preparing 4 from 3D.

Example 7

N-(2)-(allyloxyimino)methyl)-7-chloro-2,3-dihydro-4-benzofuranyl)-3,4,5,6-tetrahydrophthalimide (7)

A solution of 40 microliters of oxalyl chloride in 4 ml of methylene chloride was stirred under argon at -60°C. 65 microliters of dimethyl sulfoxide was added, and the mixture was stirred for 10 minutes. A solution of 0.1 g of 4 in 4 ml of methylene chloride was added and the mixture was stirred for 30 minutes. 0.28 ml of triethylamine was added and the mixture was allowed to warm to room temperature. 4 ml of dry pyridine and 40 mg of allyloxyamine hydrochloride were added and the mixture was stirred at room temperature for 1.5 hours. The solvents were removed by distillation with toluene; the residue was dissolved in ethyl acetate, the solution was filtered and the filtrate was chromatographed on a Chromatotron plate; by using initially a 1:9 v:v mixture of ethyl acetate and hexane as eluent and gradually increasing the ethyl acetate content; 7 was obtained, as a yellow oil.

Example 8

The p-toluenesulfonic acid ester of 4 - i.e., 8 - was prepared, as a yellow solid, m.p.: 188-190°C, by refluxing a solution of 4 and p-toluenesulfonic acid in xylene.

Example 9

N-(2-(acetoxymethyl)-7-chloro-2,3-dihydro-4-benzofuranyl)-3,4,5 6-tetrahydrophthalimide (9)

9, the acetic acid ester of 4, was obtained as a yellow glass, by treating 4 with acetic anhydride in the presence of 4-(dimethylamino)pyridine (DMAP) and pyridine.

Example 10

N-(2-carboxy-7-chloro-2,3-dihydro-4-benzofuranyl)-3,4,5, 6-tetrahydrophthalimide (10)

The alcohol, 4, was converted to the acid, 10, obtained as a beige solid, m.p.: 215-217°C, by treating the alcohol with Jones' Reagent.

Example 11

N-(2-(bromomethyl)-7-chloro-2,3-dihydro-4-benzofuranyl)-3,4,5, 6-tetrahydrophthalimide (11)

1.67 g of 4 was dissolved in a minimum amount of methylene chloride. 2.07 g of carbon tetrabromide was added, then the solution was stirred under nitrogen at 5°C while 2.0 g of triphenylphosphine was added in portions over 10 minutes. The mixture was stirred for 10 minutes in an ice bath, then for 4 hours at room temperature. The solvent was evaporated; the residue was triturated with a 9:1 v:v mixture of ether and hexane. The supernatant liquid was decanted and stripped of solvent and the residue was flash chromatographed on silica gel, by using a 1:4 v:v mixture of ethyl acetate and hexane as eluent, to give 11, as white crystals, m.p.: 134-136°C.

Example 12

N-(2-tert-butoxycarbonyl)-2,3-dihydro-7-chloro-4-benzofuranyl)-3,4,5,6-tetrahydrophthalimide (12)

0.33g of 4 was dissolved in a minimum amount of methylene chloride. 1.5 g of tert-butyl alcohol, 1.0 ml of acetic

anhydride and 0.75 g of pyridinium dichromate were added, and the mixture was stirred for 1 hour at room temperature. The mixture was flushed through silica gel, the column was rinsed with methylene chloride, the solvent was evaporated and the residue was chromatographed on silica gel, by using a 1:4 mixture of ethyl acetate and hexane as eluent, to give 12, as a yellow glass.

Example 13

The acetic acid ester of 6 - i.e., 13 - was obtained as a yellow solid, m.p.: 130-132°C, by treating 6 with acetic anhydride in the presence of DMAP and pyridine.

Examples 14 and 15

The methyl ester - i.e. 14, obtained as a yellow solid, mp.: 121-123°C - and the ethyl ester - i.e. 15, obtained as a yellow solid, m.p.: 71-76°C - of 10 were prepared by treating 10 with methanol and ethanol, respectively, in the presence of PTSA.

Examples 16 and 17

The ammonium salt - i.e., 16, obtained as a white powder, m.p.: 197-198°C (with decomposition) - and the isopropylamine salt - obtained as a tan solid, m.p.: 211-213°C (with decomposition) - of 10 were prepared by treating 10 with ammonia gas and isopropylamine, respectively.

Example 18

The sodium slat of 10 was prepared, as a yellow powder, m.p.: 280-284°C (with decomposition) by treating 10 in ethanol with a water solution of sodium bicarbonate.

Example 19

The alcohol 6 was converted to the acid, 19, obtained as a tan solid, m.p.: 229-231°C (with decomposition) by treating the alcohol with Jones' reagent.

Example 20

N-(7-chloro-2,3-dihyrdo-5-fluoro-2-methyl-4-benzofuranyl)-3,4,5,6-tetrahydrophthalimide (20)

3-(allyloxy)-4-chloro-6-fluoroaniline (20A) was prepared, as a brown oil, from 2-chloro-4-fluorophenol and allyl bromide by procedures described in U.S. patent 4,452,981.

A mixture of 9.0 g of 20A, 6.8 g of THPA and 100 ml of glacial acetic acid was heated at reflux temperature for 4 hours. The resulting mixture was cooled to room temperature, poured into 450 ml of water and extracted with ether. The extract phase was washed with water, dried ($Na_2SO_4$), and filtered, and the solvent was evaporated from the filtrate. The residue was flash-chromatographed on silica gel, with a 15:85 v:v mixture of ethyl acetate and hexane as eluent, to give N-(3-(allyloxy)-4-chloro-6-fluorophenyl)-3,4,5,6-tetrahydrophtalimide (20B), as an orange oil.

9.8 g of 20B was heated in an oil bath at 200°C for one hour. The resulting crude product was cooled to room temperature and flash chromatographed on silica gel, by using a 5:95 v:v mixture of acetone and toluene as solvent, to give N-(2-allyl-4-chloro-6-fluoro-3-hydroxyphenyl)-3,4,5,6-tetrahydrophthalimide (20C), as a tan solid, m.p.: 109-111°C.

A mixture of 80 ml of xylene and 0.5 of PTSA monohydrate was dehydrated by azeotroping for 30 minutes. The resulting solution was cooled to room temperature, 1.70 g of 20C was added and the resulting mixture was heated at reflux for 18 hours, then held at room temperature overnight. The mixture was washed with a saturated aqueous solution of sodium bicarbonate, then with water, then with brine, dried ($Na_2SO_4$), filtered and stripped of solvent. The residue was flash-chromatographed on silica gel, using a 1:4 v:v mixture of ethyl acetate and hexane as eluant, to give 20, as off-white crystals, m.p.: 169-171°C.

Example 21

N-(7-Chloro-2,3-dihydro-5-fluoro-2-(hydroxymethyl)-4-benzofuranyl)-3,4,5,6-tetrahydrophthalimide (21)

A solution of 0.67 g of 20C and 475 mg of 80% MCPBA in 15 ml of 1,2-dichloroethane was heated on an oil bath at 70°C for 2.5 hours. A few milligrams of PTSA was added, the mixture was cooled to room temperature, washed with a saturated aqueous solution of sodium sulfite, followed by a saturated aqueous solution of sodium bicarbonate, dried ($Na_2SO_4$), filtered and stripped of solvent. The residue was flash chromatographed on silica gel with a 40:60 v:v mixture of ethyl acetate and hexane as eluent, to give 21, as white crystals, m.p.: 193-195°C.

Example 22

N-(2-acetoxymethyl)-7-chloro-2,3-dihydro-5-fluoro-4-benzofuranyl)-3,4,5,6-tetrahydrophthalimide (22)

22, the acetic acid ester of 21, was obtained, as off-whtie crystals, m.p.: 129-131°C, by treating 21 with acetic anhydride in the presence of DMAP and pyridine.

Example 23

N-(2-carboxy-7-chloro-2,3-dihydro-5-fluoro-4-benzofuranyl)-3,4,5,6-tetrahydrophthalimide (23).

23 was obtained, as a white powder, m.p.: 209-211°C (with decomposition) by treating 21 with Jones' Reagent.

Example 24

N-(7-chloro-2,3-dihydro-5-fluoro-2-(methoxycarbonyl)-4-benzofuranyl)-3,4,5,6-tetrahydrophthalimide (24)

24 was prepared, as a yellow semi-solid, by treating 23 with methanol in the presence of PTSA.

Example 25

N-(7-chloro-2,3-dihydro-2-(1-ethoxycarbonyl)ethoxycarbonyl)-5-fluoro-4-benzofuranyl)-3,4,5,6-tetrahydrophthalimide (25)

A mixture of 0.300 g of 23, 120 microliters of ethyl 3-bromopropionate, 0.136 g of potassium carbonate, 0.136 g of potassium iodide and 8 ml of acetonitrile was heated at reflux for 3 hours. The resulting mixture was portioned between ether

and water; the ether phase was separated, washed with water, then brine, dried ($MgSO_4$), filtered and stripped of solvent. The residue was flash chromatographed on silica gel, by using a 1:3 v:v mixture of ethyl acetate and hexane as eluent, to give 25, as a yellow semi-solid.

Example 26

N-(7-chloro-2,3-dihydro-2-(hydroxymethyl)-4-benzofuranyl)-phthalimide (26)

26 was prepared, as a white solid, m.p.: 181-183°C, from 3B and phthalic anhydride in three steps, according to the procedures described for preparing 4 from 3D.

Example 27

N-(2-carboxy-7-chloro-2,3-dihydro-4-benzofuranyl)phthalimide (27), was prepared, as beige crystals, m.p.: 236-239°C (with decomposition) by treating 26 with Jones' Reagent.

Examples 28-31

By procedures described in the foregoing examples, the following compounds of Formula I were prepared:

28, the ethyl ester of 27, as a yellow solid, m.p.: 134-136°C;

29, the methyl ester of 27, as a yellow solid, m.p.: 136-138°C;

30, the sodium salt of 27, as a yellow solid, m.p.: not determined (decomposition above 300°C);

31, the ammonium salt of 27, as a yellow powder, m.p.: 220-223°C (with decomposition).

Example 32

N-(7-chloro-2,3-dihydro-2-methyl-4-benzofuranyl)phthalimide (32)

32 was prepared, as a beige solid, m.p.: 197-198°C, from 3B and phthalic anhydride, by the procedures described for preparing 3 from 3B.

Example 33

2-(7-Chloro-2,3-dihydro-2-methyl-4-benzofuranyl)-2,3,4-5-tetrahydro-3,5-dioxo-1,2,4-triazine-6-carbonitrile (33)

15 g of hydrazine was added to a mixture of 30 g of 32 and 120 g of dimethyl sulfoxide under nitrogen at room temperature. The mixture was stirred for 16 hours, then was added to 600 ml of water. The mixture was extracted with ether, the extract was washed with water, then with brine, dried and stripped of solvent to give 4-amino-7-chloro-2,3-dihydro-2-methylbenzofuran (33A), as a solid, m.p.: 70°C.

A mixture of 9.18 g of 33A, 10 ml of 12N hydrochloric acid and 10 ml of water was heated on a steam bath for 1 hour, then 10 ml of water was added and the mixture was stirred at room temperature overnight. Then 30 ml of water was added, the mixture was cooled to 0°C and stirred while a solution of 3.45 g of sodium nitrate in 10 ml of water was added dropwise over 15 minutes, then was stirred for 1 hour at 0°C. The resulting mixture was added slowly to a stirred mixture of 7.8 g of N-cyanoacetylurethane, 25 ml of pyridine and 900 ml of water, at 0°C. The resulting mixture was stirred at 0°C for 1.5 hours, and filtered. The collected solid phase was washed with water, dried under reduced pressure and recystallized from acetone/petroleum ether to give ethyl N-((7-chloro-2,3-dihydro-2-methyl-4-benzofuranylhydrazono)cyanoacetyl)carbamate (33B), as a solid, m.p.: 175-177°C (with decomposition).

A mixture of 12.5g of 33B and 600ml of xylene was stirred at reflux temperature overnight under a Soxhlet extractor filled with 4A molecular sieves to collect the alcohol that was formed. The mixture was cooled to room temperature and filtered; the solid product was dried under reduced pressure to give 33, as a tan solid, m.p.: 244-245°C.

Example 34

2-(7-Chloro-2,3-dihydro-2-methyl-4-benzofuranyl)-2,3,4,5-tetrahydro-3,5-dioxo-1,2,4-triazine-6-carboxylic acid (34)

A mixture of 9.0 g of 33, 150 ml of p-dioxane and 100 ml of 6N hydrochloric acid was stirred at reflux temperature overnight. The 20 ml of 12N hydrochloric acid was added and the mixture was stirred at reflux temperature for 2 hours, and

stripped of solvent. The residue was dissolved in ethyl acetate, the solution was washed with water, then brine, dried ($MgSO_4$) and stripped of solvent. The residue was recrystallized from ethyl acetate/petroleum ether to give 34, as an amber solid, m.p.: 235-236°C (with decomposition0.

Example 35

2-(7-Chloro-2,3-dihydro-2-methyl-4-benzofuranyl)-1,2,4-triazine-3,5(2H,4H)-dione (35)

A mixture of 8.0 g of 34 and 80 ml of Dowtherm was heated at 200-250°C for 2 hours, cooled to room temperature, diluted with petroleum ether and filtered. The collected solid phase was dried under reduced pressure, and recystallized from ethyl acetate/petroleum ether to give 35, as a tan solid, m.p.: 184-186°C.

Example 36

2-(7-Chloro-2,3-dihydro-2-methyl-4-benzofuranyl)-4-methyl-3,5-dioxo-1,2,4-triazine-3,5(2H,4H)-dione (36)

2.8 g of 35 was added over 15 minutes to a stirred mixture of 0.5 g of hexane-washed sodium hydride, 1.6 g of methyl iodide and 30 ml of dry DMF, under nitrogen at 0°C. When gas evolution ended (30 minutes) the mixture was stirred at room temperature for 1.5 hours, poured into ice-water and the resulting mixture was filtered. The collected solid was dissolved in ethyl acetate; the resulting solution was washed with water, then brine, dried ($MgSO_4$) and stripped of solvent. The residue was recystallized from ethyl acetate/hexane to give 36, as a tan solid, m.p.: 131-132°C.

Examples 37 and 38

2-(7-Chloro-2,3-dihydro-2-methyl-4-benzofuranyl)-4-(difluoromethyl)-5-methyl-2H-1,2,4,-triazol-3(4H)-one (37);

2-(7-chloro-2,3-dihydro-2-methyl-4-benzofuranyl)-1-(difluoromet-yl)-5-methyl-1H-1,2,4-triazol-3(2H)-one (38)

5.5 g of 33A was diazotized by the procedure described in Example 33. The diazotization mixture was cooled to -25°C and a solution of 19.7 g of tin (II) chloride dihydrate in 25 ml of

12N hydrochloric acid was added dropwise over 20 minutes to the stirred mixture, which was stirred thereafter at -25°C for 4 hours. Then a solution of 2.82 g of redistilled pyruvic acid in 20 ml of water was added dropwise to the stirred mixture at -25°C, then while being stirred was allowed to warm slowly to room temperature and stirred over a weekend. Then the mixture was filtered, and the solid material was washed with water and dissolved in a saturated aqueous solution of sodium bicarbonate. The solution was extracted with ethyl acetate, and the aqueous phase was acidified with 12N hydrochloric acid and extracted with ethyl acetate. The latter extract was washed with water, then with brine, dried ($MgSO_4$) and stripped of solvent. The residue was recystallized from ethyl acetate/hexane to give 2-((7-chloro-2,3-dihydro-2-methyl-4-benzofuranyl)hydrazono)propanoic acid (37A), a mixture of isomers, m.p.: 155-157°C.

5.1 g of triethylamine was added to a stirred mixture of 5.0 g of 37A and 100 ml of toluene, then 1.9 g of diphenylphosphoryl azide was added and over two hours the mixture was heated to and held at reflux temperature. The resulting mixture was heated to and held at reflux temperature. The resulting mixture was poured into 100 ml of 10% w:v aqueous ice-cold potassium hydroxide solution. Two liquid phases resulted and were separated. The aqueous phase was acidified with 12N hydrochloric acid, and extracted with ethyl acetate. The extract was washed with water, then with brine, dried ($MgSO_4$) and stripped of solvent. The residue was recrystallized from ethyl acetate/hexane to give 2-(7-chloro-2,3-dihydro-2-methyl-4-benzofuranyl)-5-methyl-2H- 1-,2,4-triazol-3(4H)-one (37B), as a colourless solid, m.p.: 208-209°C.

A mixture of 0.6 g of hexane-washed sodium hydride and 30 ml of THF was saturated with Freon 22 (chlorodifluoromethane) at 0°C, then a mixture of 1.33 g of 37B and 10 ml of THF was added and the mixture was stirred at room temperature for 6 hours, Freon 22 being bubbled into the mixture throughout that

period. The mixture was stirred at room temperature for a week, 15 ml of water was added dropwise, and the solvent was stripped. The residue was dissolved in ethyl acetate, the solution was washed with water, then with brine, dried ($MgSO_4$) and stripped of solvent. The residue was chromatographed on silica gel, with a 1:3 v:v mixture of hexane and methylene chloride as eluent to give two fractions, yielding two isomeric products: 37, as a colourless solid, m.p.: 122.5-123.5°C, and 38 as a cream-coloured solid, m.p.: 78-79°C.

Example 39

2-(7-Chloro-2,3-dihydro-2-methyl-4-benzofuranyl)hexahydro-1H-imidazo[3,4-a]pyridine-1,3(2H)-dione (39)

6.07 g of triethylamine was added to a mixture of 9.18 g of 33A, 11.87 g of trichloromethyl chloroformate and 250 ml of dioxane, and the mixture was refluxed for 6 hours. The mixture was filtered, and the filtrate was stripped of solvent, to give 7-chloro-4-isocyanato-2-methylbenzofuran (39A), as a brown liquid.

A solution of 2.36 g of ethyl pipecolinate in 20 ml of dry THF was added, at room temperature under nitrogen, to a mixture of 2.10 g of 39A and 60 ml of dry THF, the rate of addition being such as to maintain the temperature of the mixture at 25-32°C, then the mixture was stirred overnight at room temperature, diluted with 250 ml of hexane and filtered. The collected solid phase was identified as ethyl 1-(((7-chloro-2,3-dihydro-2-methyl -4-benzofuranyl)-amino)carbonyl)-2-piperidinecarboxylate (39B), m.p.: 169-171°C.

A mixture of 2.0 g of 39B, 25 ml of ethanol and 25 ml of 2N hydrochloric acid was stirred and refluxed for 2 hours. Then the solvent was evaporated, the residue was mixed with 50 ml of water, and the resulting mixture was extracted with methylene chloride. The extract was dried ($MgSO_4$) and the solvent was evaporated. The residue was purified by filtration through a

short pad of silica gel, with a 1:1 v:v mixture ethyl acetate and hexane as eluent, to give 39, as a solid, m.p.: 145-148°C.

Example 40

3-(7-Chloro-2,3-dihydro-2-methyl-4-benzofuranyl)-1-methylimidazolidine-2,4-dione (40)

2.30 g of sarcosine ethyl ester hydrochloride was added over 5 minutes to a mixture of 2.10 g of 39A, 1.52g of triethylamine and 60 ml of methylene chloride, under nitrogen at 25-30°C. The mixture was stirred at room temperature for 24 hours, then diluted with 100 ml of methylene chloride, washed with water, then brine, dried ($MgSO_4$) and stripped of solvent. The residue was dissolved in 70 ml of a 1:1 v:v mixture of ethanol and 2N hydrochloric acid. The solution was refluxed for 4 hours, the resulting mixture was stirred at room temperature overnight, stripped of volatiles, diluted with water and extracted with methylene chloride. The extract was dried ($MgSO_4$) and stripped of solvent. The residue was purified by flash chromatography over 56-60 mesh silica gel with a 1:2 v:v mixture of hexane and ethyl acetate as eluent to give 40, as a glass/sticky solid.

Example 41

Herbicidal Activity

In the following examples, the species of plants that were tested were:

| | Abbreviation |
|---|---|
| Barnyardgrass - *Echinochloa crus-galli* | BYGR |
| Downy brome - *Bromus tectorum* | DOBR |
| Yellow foxtail - *Setaria glauca* | YEFT |
| Sicklepod - *Cassia obtusifolia* | SIPO |
| Velvetleaf - *Abutilon theophrasti* | VELE |
| Garden cress - *Lepidium sativum* | GACR |
| Johnsongrass - *Sorghum halepense* | JOGR |
| Morninglory - *Ipomoea sp.* | MOGL |
| Field bindweed - *Convolvulus arvensis* | FIBW |
| Nightshade - *Solanum sp.* | NISH |

| | | |
|---|---|---|
| Blackgrass - Alopecurus myosuroides | | BLGR |
| Yellow millet - Panicum miliceum | | YEMI |
| Large crabgrass - Digitaria sanguinalis | | LACG |
| Redroot pigweed - Amaranthus retroflexus | | RRPW |
| Hemp sesbania - Sesbania exaltata | | HESE |
| Prickly sida - Sida spinosa | | PRSI |

Test Procedures

The pre-emergence (soil) herbicidal activity of compounds of Formula I was evaluated by planting seeds of downy brome, johnsongrass, yellow foxtail, barnyardgrass, yellow millet, blackgrass, hemp sesbania, velvetleaf, morningglory, prickly sida, sicklepod and garden cress in test tubes, nominally measuring 25 x 200 millimeters, filled about three-quarters full of untreated soil, in each case covered on top with about 2.5 cubic centimetres of soil, treated with 0.1 milligram of the test compound, to give a dosage of 1.12 kg/ha of test compound (1.0 pound per acre). The seeds were planted on top of the treated soil and covered with about 1.5 cubic centimetres of untreated soil. The planted soil was held under a controlled regimen of temperature, moisture, and light. At 10 days, the amounts of germination and growth in each tube were evaluated on a 0 to 9 scale, the numeric ratings having the following meanings:

| Rating | Meaning |
|---|---|
| 9 | No living tissue |
| 8 | Plant severely damaged and expected to die |
| 7 | Plant badly damaged, but expected to live |
| 6 | Moderate damage, but complete recovery expected |
| 5 | Intermediate damage (probably unacceptable for crop plants) |
| 3-4 | Observable damage |
| 1-2 | Plant slightly affected, possibly by the chemical, possibly due to biological variability |
| 0 | No visible effect |

The postemergence (foliar) herbicidal activity of compounds of Formula I was evaluated by spraying 9-day-old large crabgrass plants, 9-day-old pigweed plants, 6-day-old johnsongrass plants, 9-day-old velvetleaf plants, 8-day-old yellow foxtail plants, 9-day-old sicklepod plants, 5-day-old morninglory plants, 5-day-old barnyardgrass plants, 6-day-old yellow millet plants, 9-day-old nightshade plants, 9-day-old prickly sida plants and 7-day-old field bindweed plants to runoff with 2.4 millilitres of a liquid formulation containing 0.5 milligram of the test compound (1.12 kg of the test compound per hectare). The sprayed plants were held under a controlled regimen of temperature, moisture and light for 7 to 8 days, when the effect of the test compound was evaluated visually, the results being rated on the 0 to 9 scale described above.

Results of the pre-emergence and postemergence herbicidal activity tests are set forth in Tables I and II.

TABLE I. PRE-EMERGENCE HERBICIDAL ACTIVITY

| Compound No. | DOBR | JOGR | YEFT | BYGR | YEMI | BLGR | HESE | VELE | MOGL | PRSI | SIPO | GACR |
|---|---|---|---|---|---|---|---|---|---|---|---|---|
| 1 | 0 | 0 | 0 | 0 | 0 | 0 | 0 | 0 | 0 | 0 | 0 | 4 |
| 2 | 0 | 6 | 3 | 7 | 5 | 0 | 0 | 0 | 0 | 6 | 0 | 5 |
| 3 | 4 | 6 | 4 | 6 | 0 | 7 | 8 | 9 | 4 | 9 | 0 | 9 |
| 4 | 6 | 7 | 4 | 7 | 7 | 7 | 8 | 8 | 8 | 9 | 3 | 9 |
| 5 | 0 | 0 | 0 | 0 | 0 | 0 | a) | 3 | 0 | 3 | 0 | 9 |
| 6 | 5 | 6 | 6 | 6 | 6 | 5 | 8 | 6 | 6 | 8 | 3 | 9 |
| 7 | 0 | 3 | 0 | 4 | 0 | 4 | 5 | 7 | 0 | 8 | 0 | 6 |
| 9 | 7 | 7 | 6 | 8 | 7 | 6 | 8 | 9 | 9 | 9 | 7 | 9 |
| 10 | 5 | 3 | 2 | 3 | 3 | 2 | 8 | 9 | 9 | 8 | 2 | 9 |
| 11 | 3 | 0 | 0 | 5 | 0 | 0 | 8 | 9 | 3 | 4 | 0 | 9 |
| 12 | 3 | 0 | 0 | 5 | 0 | 3 | 3 | 7 | 5 | 9 | 0 | 9 |
| 13 | 5 | 4 | 5 | 6 | 6 | 3 | 7 | 7 | 3 | 8 | 0 | 9 |
| 14 | 5 | 4 | 5 | 6 | 0 | 3 | 4 | 9 | 9 | 9 | 0 | 9 |
| 15 | 3 | 0 | 4 | 4 | 3 | 0 | 3 | 9 | 9 | 9 | 3 | 9 |
| 16 | 5 | 3 | 4 | 5 | 0 | 3 | 0 | 9 | 9 | 9 | 0 | 9 |
| 17 | 5 | 3 | 3 | 5 | 3 | 3 | 0 | 9 | 9 | 9 | 3 | 9 |
| 19 | 0 | 0 | 0 | 0 | 0 | 0 | 0 | 4 | 6 | 3 | 0 | 6 |

| Compound No. | DOBR | JOGR | YEFT | BYGR | YEMI | BLGR | HESE | VELE | MOGL | PRSI | SIPO | GACR |
|---|---|---|---|---|---|---|---|---|---|---|---|---|
| 20 | 5 | 6 | 9 | 9 | 7 | 7 | 9 | 9 | 6 | 9 | 8 | 9 |
| 21 | 5 | 6 | 7 | 7 | 9 | 7 | 9 | 9 | 9 | 9 | 9 | 9 |
| 22 | 9 | 6 | 8 | 8 | 9 | 7 | 9 | 9 | 9 | 9 | 9 | 9 |
| 23 | 7 | 5 | 5 | 7 | 8 | 3 | 9 | 9 | 9 | 9 | 0 | 9 |
| 24 | 7 | 3 | 7 | 9 | 9 | 3 | 9 | 9 | 9 | 9 | 0 | 9 |
| 26 | 0 | 5 | 3 | 3 | 0 | 3 | 3 | 9 | 8 | 9 | 0 | 9 |
| 27 | 3 | 3 | 4 | 4 | 3 | 3 | 3 | 9 | 7 | 9 | 0 | 9 |
| 28 | 0 | 0 | 0 | 0 | 0 | 0 | 0 | 3 | 5 | 8 | 0 | 8 |
| 29 | 0 | 0 | 0 | 0 | 0 | 0 | 2 | 5 | 4 | 8 | 2 | 9 |
| 30 | 0 | 0 | 5 | 8 | 0 | 0 | 0 | 9 | 8 | 9 | 0 | 9 |
| 31 | 0 | 4 | 6 | 5 | 6 | 0 | 0 | 9 | 9 | 9 | 3 | 8 |
| 32 | 0 | 0 | 0 | 0 | 0 | 0 | 0 | 0 | 0 | 0 | 0 | 0 |

TABLE II. POSTEMERGENCE HERBICIDAL ACTIVITY

| Compound No. | LACG | JOGR | YEFT | BYGR | YEMI | RRPW | NISH | VELE | MOGL | PRSI | SIPO | FIBW |
|---|---|---|---|---|---|---|---|---|---|---|---|---|
| 1 | 3 | 4 | 3 | 3 | 5 | 3 | 9 | 4 | 8 | 5 | 3 | 5 |
| 2 | 3 | 3 | 3 | 3 | 5 | 7 | 9 | 7 | 8 | 6 | 3 | 9 |
| 3 | 8 | 9 | 9 | 9 | 9 | 9 | 9 | 9 | 9 | 9 | 9 | 9 |
| 4 | 5 | 9 | 9 | 8 | 8 | 9 | 9 | 9 | 9 | 9 | 8 | 9 |
| 5 | 2 | 7 | 7 | 7 | 7 | 6 | 8 | 7 | 9 | 8 | 7 | 7 |
| 6 | 3 | 5 | 5 | 3 | 3 | 7 | 9 | 5 | 9 | 6 | 0 | 9 |
| 7 | 7 | 9 | 9 | 9 | 9 | 9 | 9 | 9 | 9 | 9 | 8 | 9 |
| 9 | 7 | 6 | 9 | 6 | 7 | 9 | 9 | 9 | 9 | 9 | 9 | 9 |
| 10 | 3 | 5 | 9 | 3 | 4 | 9 | 9 | 9 | 9 | 9 | 3 | 9 |
| 11 | 6 | 9 | 9 | 9 | 9 | 9 | 9 | 9 | 9 | 9 | 9 | 9 |
| 12 | 8 | 9 | 9 | 9 | 9 | 9 | 9 | 9 | 9 | 9 | 9 | 9 |
| 13 | 3 | 4 | 5 | 3 | 3 | 5 | 9 | 3 | 8 | 7 | 3 | 9 |
| 14 | 6 | 9 | 9 | 9 | 9 | 9 | 9 | 9 | 9 | 9 | 7 | 9 |
| 15 | 6 | 9 | 9 | 6 | 7 | 9 | 9 | 9 | 9 | 9 | 8 | 9 |
| 16 | 6 | 8 | 9 | 6 | 6 | 9 | 9 | 9 | 9 | 9 | 6 | 9 |
| 17 | 5 | 8 | 9 | 6 | 7 | 9 | 9 | 9 | 9 | 9 | 3 | 9 |
| 19 | 4 | 7 | 6 | 3 | 5 | 9 | 9 | 6 | 9 | 8 | 4 | 9 |

| Compound No. | LACG | JOGR | YEFT | BYGR | YEMI | RRPW | NISH | VELE | MOGL | PRSI | SIPO | FIBW |
|---|---|---|---|---|---|---|---|---|---|---|---|---|
| 20 | 9 | 9 | 9 | 9 | 9 | 9 | 9 | 9 | 9 | 9 | 9 | 9 |
| 21 | 7 | 9 | 9 | 9 | 7 | 9 | 9 | 8 | 9 | 9 | 9 | 9 |
| 22 | 7 | 8 | 9 | 9 | 7 | 9 | 9 | 9 | 9 | 9 | 9 | 9 |
| 23 | 9 | 5 | 9 | 9 | 8 | 9 | 9 | 9 | 9 | 9 | 7 | 9 |
| 24 | 7 | 7 | 9 | 9 | 7 | 9 | 9 | 9 | 9 | 9 | 7 | 9 |
| 25 | 5 | 9 | 9 | 9 | 8 | 9 | 9 | 9 | 9 | 9 | 6 | 9 |
| 26 | 3 | 2 | 2 | 3 | 3 | 6 | 7 | 3 | 9 | 6 | 4 | 9 |
| 27 | 8 | 9 | 9 | 9 | 8 | 9 | 9 | 9 | 9 | 9 | 3 | 9 |
| 28 | 6 | 5 | 8 | 7 | 6 | 9 | 9 | 8 | 9 | 9 | 5 | 0 |
| 29 | 8 | 9 | 9 | 8 | 8 | 9 | 9 | 9 | 9 | 9 | 4 | 9 |
| 30 | 8 | 9 | 8 | 7 | 7 | 9 | 9 | 8 | 9 | 9 | 7 | 9 |
| 31 | 7 | 8 | 9 | 8 | 8 | 9 | 9 | 7 | 9 | 9 | 6 | 9 |
| 32 | 5 | 3 | 4 | 3 | 4 | 5 | 9 | 5 | 6 | 6 | 4 | 5 |

PS05006

K 3609 FF

CLAIMS

1. A compound having one of the formula I:

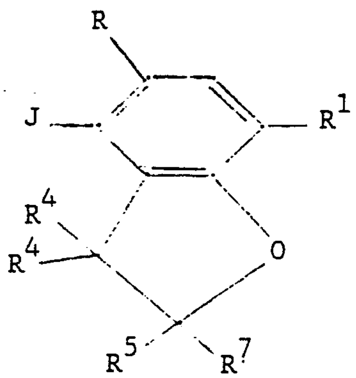

A

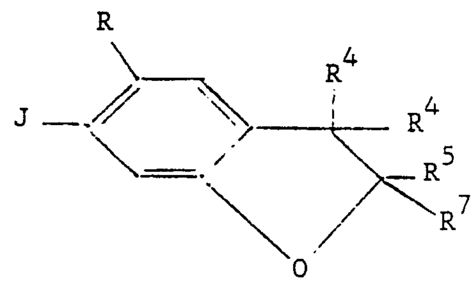

B

(I)

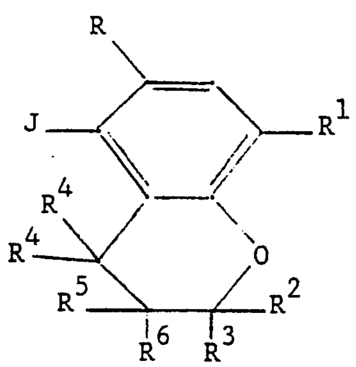

C

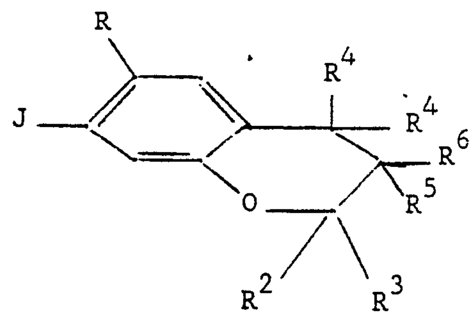

D

wherein
R and $R^1$ each independently represents a hydrogen or a halogen atom; a cyano group; an alkyl or alkoxy group having one to four carbon atoms; a trihalomethyl group; a

hydroxymethyl group; an alkyl-, alkenyl-, or alkanoyl-oxymethyl group having up to four carbon atoms; or $-OCF_nH_{3-n}$ where n is one, two or three;

each of $R^4$ is independently a hydrogen atom or an alkyl group having one to four carbon atoms;

$R^5$ represents a hydrogen atom or an alkyl group having one to four carbon atoms;

$R^6$ is a hydrogen atom or a hydroxy group; with the proviso that $R^5$ and $R^6$ together can be an oxo (=O) oxygen atom or a moiety $=NO(R^8)$; and with the further proviso that when $R^5$ is a hydrogen atom, $R^6$ is -CN, -C(O)Cl, $-C(O)R^8$, $-C(O)O(R^8)$, or $-C(O)N(R^8)(R^8)$;

$R^7$ represents a group -CN, $-CH(R^9)CN$, $-CH(R^2)(R^3)$, -C(O)Cl, $-C(O)R^8$, $-C(O)O(R^8)$, $-CH(R^9)-O(R^{10})$, $-C(O)N(R^8)(R^8)$ or $-C(R^9)=NO(R^8)$, wherein $R^2$ and $R^3$ each independently represents a hydrogen atom; an alkyl or haloalkyl group of one to four carbon atoms; a phenyl group; or a phenylalkyl group having seven to ten carbon atoms;

$R^8$ represents a hydrogen atom; an alkyl, haloalkyl, alkenyl, haloalkenyl or alkynyl group of up to four carbon atoms; a phenyl or phenylalkyl group having seven to ten carbon atoms; a heteroaryl or heteroaralkyl group having up to ten carbon atoms; or an alkoxyalkyl or alkoxycarbonylalkyl group having up to six carbon atoms;

$R^9$ represents a hydrogen atom or an alkyl group having one to four carbon atoms;

$R^{10}$ represents a hydrogen atom; an alkyl, haloalkyl, alkenyl, haloalkenyl or alkynyl group having up to four carbon atoms; a phenylalkyl group having seven to ten carbon atoms; a heteroaralkyl group having up to ten carbon atoms; or each of the foregoing bonded to a carbonyl moiety that is bonded to the oxygen atom; an alkoxyalkyl or alkoxycarbonylalkyl group having up to six carbon atoms; an alkylsulfonyl group having one to four carbon atoms; a phenylsulfonyl group which may be substituted on the ring

by one or more halogen atoms and/or alkyl groups having one to four carbon atoms; a carbamoyl group, or an alkyl- or dialkylcarbamoyl group having up to six carbon atoms; and J is one of the moieties:

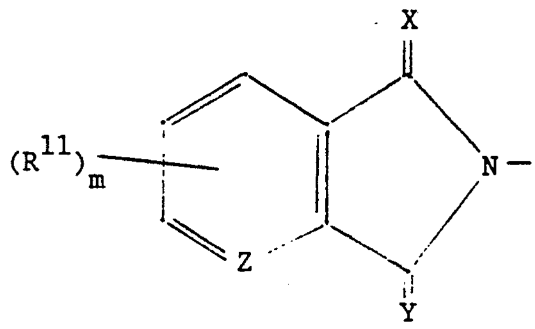

J-1

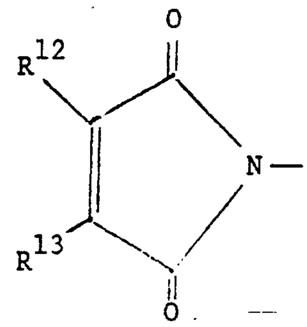

J-2

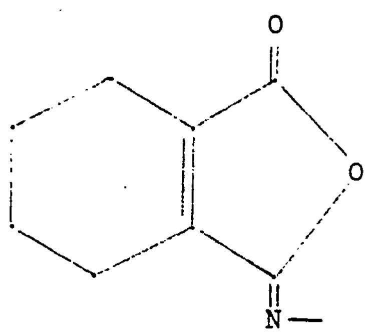

J-3

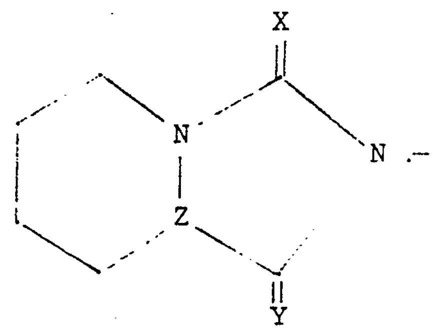

J-4

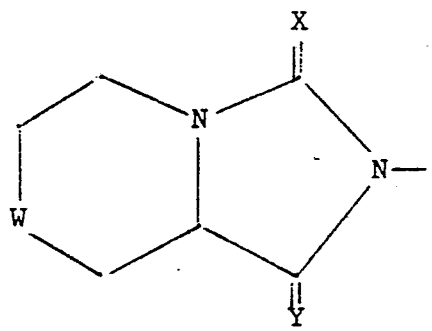

J-5

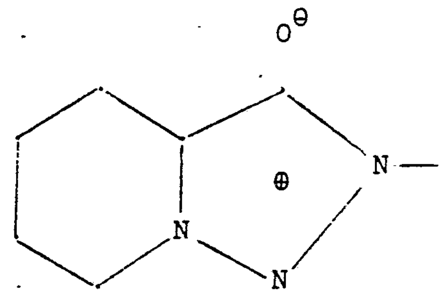

J-6

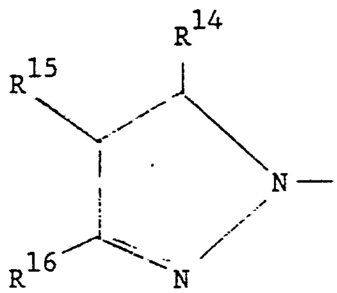

J-7

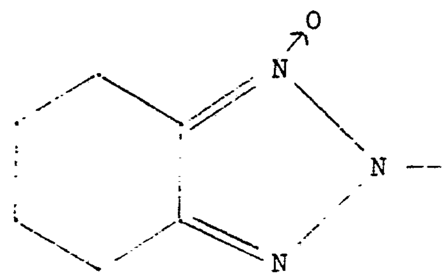

J-8

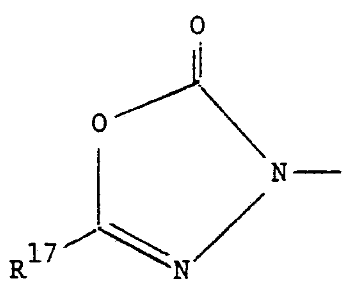

J-9

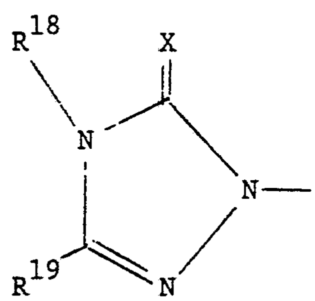

J-10

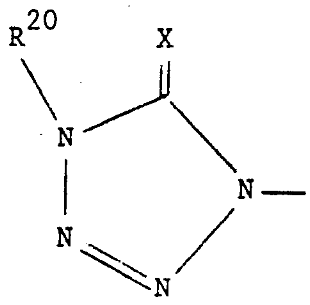

J-11

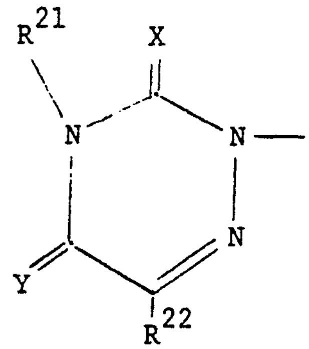

J-12

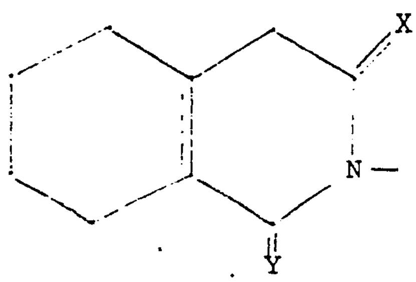

J-13

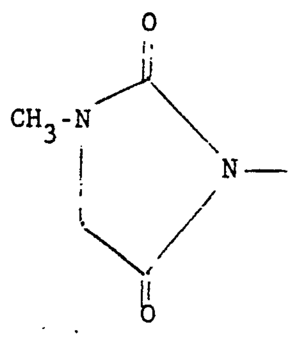

J-14

wherein

X and Y each is O or S;

Z is C or N;

W is S or $SO_2$;

m is zero, one or two;

$R^{11}$ is a halogen atom, or is an alkyl, haloalkyl or alkoxy group having one to four carbon atoms:

$R^{12}$ and $R^{13}$ each independently represents a hydrogen atom or an alkyl group having one to four carbon atoms, or $R^{12}$ and $R^{13}$ together with the carbon atoms to which they are attached form an alicyclic six-membered ring that may be substituted by one to three methyl groups;

$R^{14}$ is a hydrogen or halogen atom, or an alkyl, or alkyl-$S(O)_a$- group having one to four carbon atoms wherein a is zero, one or two;

$R^{15}$ is a hydrogen or halogen atom; or an alkyl, alkylthio or alkylsulfonyl group having one to four carbon atoms;

$R^{16}$ is an alkyl group having one to four carbon atoms, or $R^{15}$ and $R^{16}$ together form the moiety, $-(CH_2)_b-$, wherein b is three or four, which may be substituted by one to three methyl groups;

$R^{17}$ is a hydrogen atom or an alkyl, alkenyl, haloalkyl or alkoxyalkyl group having up to six carbon atoms, or a cycloalkyl group having three to six carbon atoms, a cycloalkylalkyl group having four to eight carbon atoms, or either of these groups substituted by one to three alkyl groups each having one to four carbon atoms;

$R^{18}$ has one to six carbon atoms and is an alkyl, haloalkyl, cyanoalkyl, alkoxyalkyl, alkenyl, alkynyl, or -alkyl-$S(O)_a$-alkyl group where a is zero, one or two;

$R^{19}$ is a hydrogen atom, a group $R^{18}$ as defined above, or $R^{18}$ and $R^{19}$ together represent an alkylene moiety $-(CH_2)_b-$, as defined above for $R^{16}$, or a moiety $-(CH_2)_c-U-(CH_2)_d-$ wherein c and d each is zero, one, two or three, and c and d = two or three, and U is oxygen, $-S(O)_a-$ or -NR alkyl- where a is zero, one or two, with the proviso that when U is $-S(O)_a-$, c is other than zero;

$R^{20}$ has one to six carbon atoms, and is an alkyl, haloalkyl, alkoxyalkyl, alkylthioalkyl, cyanoalkyl, haloalkoxyalkyl, alkenyl and haloalkenyl group;

$R^{21}$ is a hydrogen atom, or has one to four carbon atoms and is an alkyl, haloalkyl, cyanoalkyl, alkenyl, alkynyl, alkoxyalkyl, amino, or alkoxycarbonyl group;

$R^{22}$ is a group $R^{20}$ as defined above, or is a carboxyl or alkoxycarbonyl group;

or J is benzhydrylamino, benzoylamino, acetylamino, benzylamino, allylamino, trichloroacetylamino, amino or isocyanato;

and salts of the acidic species with alkali metal bases, ammonia and amines.

2. A compound of formula IA as defined in claim 1 wherein J is J-1 or J-2.

3. A compound according to claim 2 wherein J is J-1 and m is 0 or wherein J is J-2 and $R^{12} + R^{13}$ is $-(CH_2)_4-$.

4. A compound according to claim 1, 2 or 3 wherein $R^1$ is halogen.

5. A process for the preparation of a compound of one of the formulae I as defined in claim 1 comprising cyclising, either directly or via an epoxide intermediate, a compound of one of the formulae II

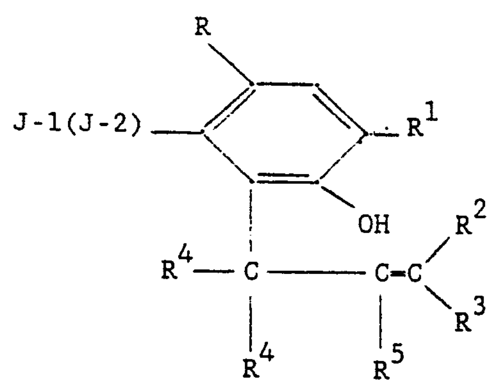

(A)

and

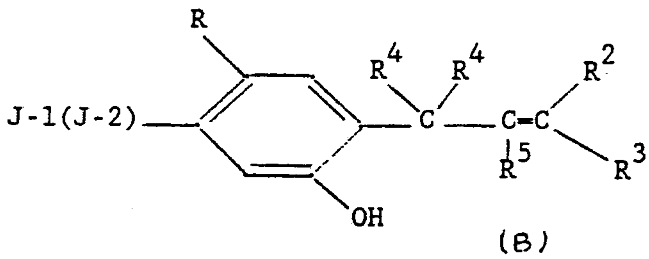

(B) (II)

where J is J-1, J-2 or an amino or substituted amino group, to give a compound of one of formulae I where J is J-1, J-2 or an amino or substitued amino group, $R^7$ is $-CH(R^2)(R^3)$ and $R^6$ is a hydrogen atom; optionally further reacting that compound of formula I where $R^7$ is $-CH(R^2)(R^3)$ and $R^6$ is a hydrogen atom to give further compounds of formula I where $R^6$ and $R^7$ have alternative meanings as defined in claim 1; and/or, where J is an amino or substituted amino group, subsequently reacting the amino group, optionally after derivatization, with a suitable reagent to provide a group J as defined for any one of J-1 to J-14; and optionally when X and/or Y is oxygen, converting the oxygen to sulphur.

6. A compound having one of the formulae I as defined in claim 1 when prepared by the process of claim 5.

7. A compound of one of the formulae II(A) and (B), III(A) and (B) or IV(A), (B), (C) or (D).

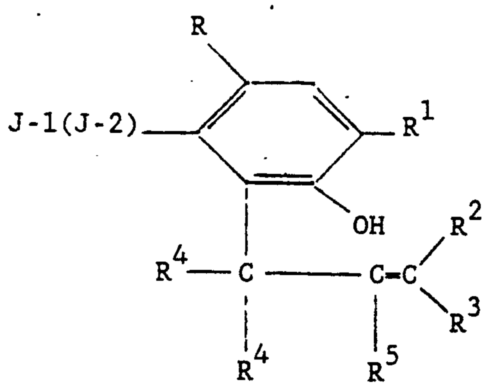

(A)

and

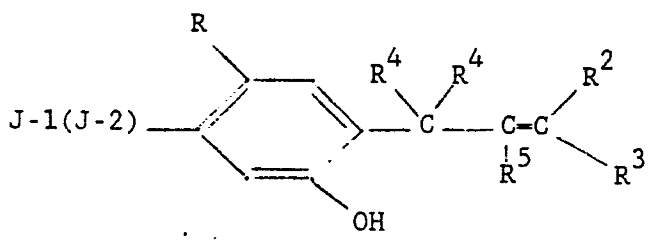

(II)

(B)

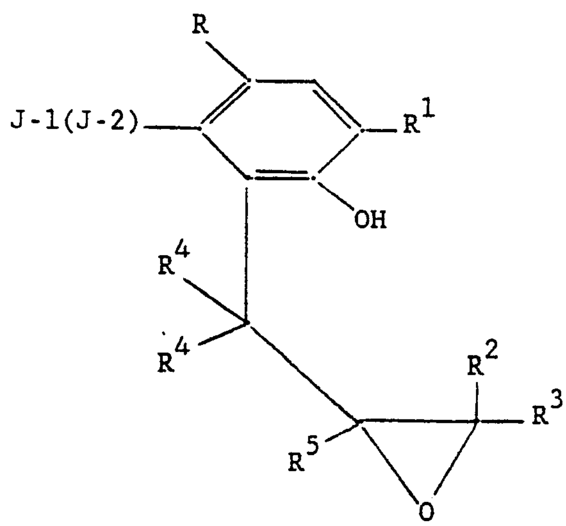

(A)

and

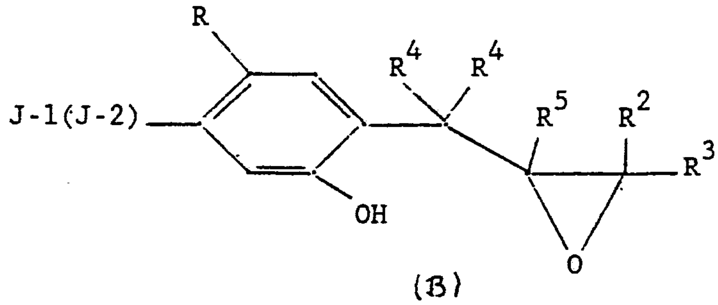

(B)

(III)

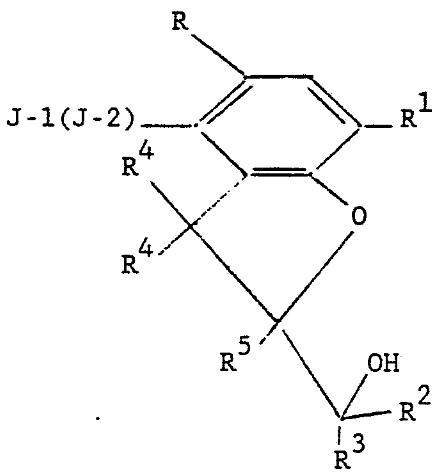

(A)

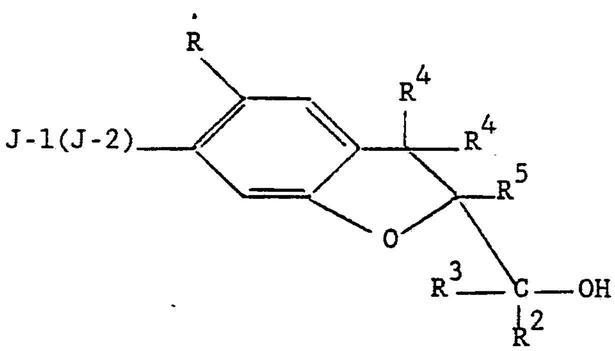

(B)

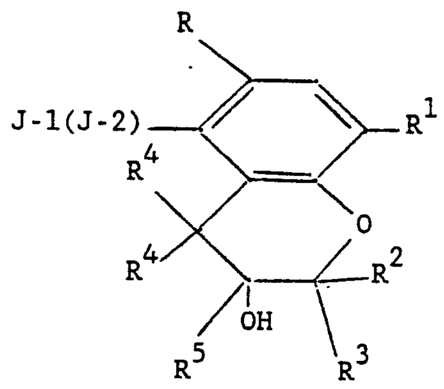

(C)

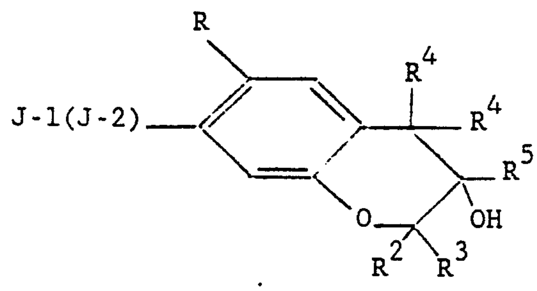

(D) (IV)

wherein the symbols have the respective meanings set forth in claim 1.

8. A process for the preparation of a compound of formula II as defined in claim 7 conprising subjecting a compound of formula V

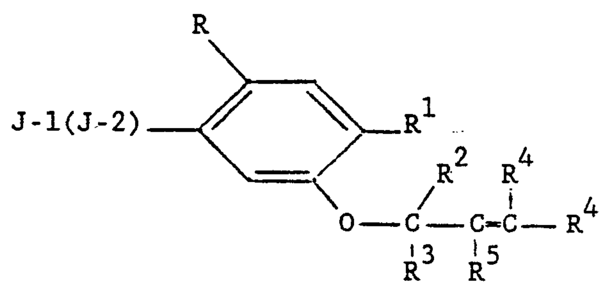

(V)

where R, $R^1$ to $R^5$, J-1 and J-2 are as defined in claim 1 to conditions effecting rearrangement; the compound of formula V having optionally been obtained by reacting a compound of formula VI:

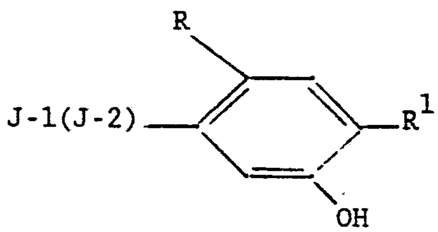

(VI)

where R, $R^1$, J-1 and J-2 are as defined in claim 1, with an allyl halide of formula VII

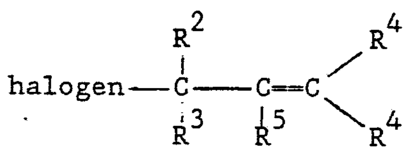

(VII)

where $R^2$, $R^3$, $R^4$ and $R^5$ are as defined in claim 1 or by heating an allyloxyaniline of formula IX

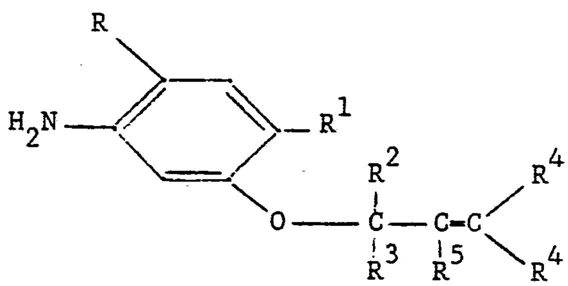

(IX)

where R and $R^1$ to $R^5$ are as defined in claim 1 with an anhydride capable of introducing a group J-1 or J-2; the compound of formula VI having optionally been obtained by heating an aniline of formula (VIII)

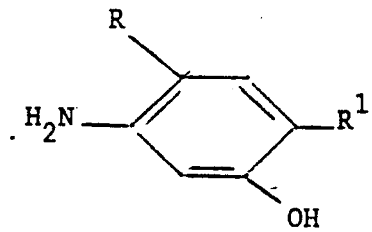

(VIII)

where R and $R^1$ are as defined in claim 1 with a phthalic, 3,4,5,6-tetrahydropthalic, quinolinic or maleic anhydride.

9. A compound of formula V, VI or VIII as defined in claim 8.

10. A herbicidal composition, which comprises a compound according to any one of claims 1 to 4 or 6, together with at least one carrier.

11. A method combatting undesired plant growth at a locus, which comprises reacting the locus with a compound as claimed in any one of claims 1 to 4 or 6, or with a composition as claimed in claim 10.

12. The use of a compound as claimed in any one of claims 1 to 4 or 6 or a composition as claimed in claim 10 as a herbicide.